# EUROPEAN PATENT APPLICATION

(11) **EP 1 507 237 A2**
(43) Date of publication of application: **16.02.2005**
(21) Application number: 04018273.5
(22) Date of filing: 02.08.2004
(51) Int. Cl.: G06T 17/40, G06T 11/20

(54) **Manipulating biological data**

(30) Priority: 14.08.2003 US 641492
(71) Applicant: Agilent Technologies Inc. (a Delaware Corporation), Palo Alto, CA 94306-2024 (US)
(72) Inventor: Kuchinsky, Allan J., San Francisco, CA 94127 (US); Vailaya, Aditya, Santa Clara, CA 95054 (US); Kincaid, Robert, Half Moon Bay, CA 94019 (US); Bluvas, Peter, Amsterdam, NY 12010 (US); Adler, Annette, Palo Alto, CA 94301 (US); Moh, David, San Francisco, CA 94131 (US); Myerholtz, Carl, Cupertino, CA 95014 (US)
(74) Representative: Schoppe, Fritz, Dipl.-Ing.

(57) **Abstract**

Systems, methods and recordable media for interactively importing, creating, and manipulating biological diagrams. Such diagrams (100,300, 511,512,513,514,515,516) maybe used for linking and navigating to other sources of biological information. Such diagrams (100,300, 511,512,513,514,515,516) may also be used interactively with other diagrams or other views of biological knowledge

## Description

### FIELD OF THE INVENTION

The present invention pertains manipulation of biological data. More particularly, the present invention pertains to systems, methods and recordable media for interactively importing, creating and/or manipulating biological diagrams.

### COPYRIGHT NOTICE

Contained herein is material that is subject to copyright protection. The copyright owner has no objection to the facsimile reproduction of the patent disclosure by any person as it appears in the Patent and Trademark Office patent files or records, but otherwise reserves all rights to the copyright whatsoever.

### BACKGROUND OF THE INVENTION

The completion of mapping of the human genome in 2000, has led to an increased focus on functional genomics, i.e., extracting functional knowledge regarding various biological processes. Various experimental methods and tools are being invented to shed light into the functioning of biological processes, at a molecular level, within various organisms, with the final goal being to understand these in humans. A common way to represent the known functional biological knowledge is via pathway diagrams, cellular networks, and diagrams of biological and chemical models.

These representations are used to display information such as signal transduction pathways, regulatory pathways, metabolic pathways, protein-protein interactions, etc. These diagrams represent biological relationships (such as bind, cleave, inhibit, promote, catalyze, etc.) between entities (genes, proteins, mRNA, other biomolecules, chemical compounds other molecules of interest) along with their localization within the cell, tissue, or organism. These visual representations are graphical in nature and are static images, i.e., they cannot be revised, supplemented or otherwise edited. Hence, they present the results for human visualization, rather than in a machine interpretable format.

Biological diagrams, such as a biochemical pathway chart, are familiar and useful reference tools for biologists, for standard representations of biochemical pathways that are analogous to representation of chemicals on the Periodic Chart. Thus, a significant aspect of the value of standard biological diagrams is that they are universally taught and understood by many bioscientists. For this reason, they also lend themselves well as a means for collaboration among scientists. An example of their use in collaboration, is that data from various sources may be overlaid upon an existing diagram for comparison purposes. It is often quite useful to be able to view experimental data in the context of a biological diagram that represents a pathway or network. Marking data of interest in a biological diagram is a very laborious and error-prone task, but is frequently performed by biologists out of necessity. When this marking task is performed manually, it is often done on a laminated chart with colored markers. This is cumbersome, time consuming and unwieldy, since changes are difficult to make, and the information generated is not easily transferable to others. There is a need for more automated tools for using and interacting with biological diagrams.

Various attempts at techniques for augmenting or replacing the need to manually generate biological diagrams have been proposed, with limited functionality. GenMapp (http://www.genmapp.org) is a free computer application designed to visualize gene expression data on maps representing biological pathways and groupings of genes. The only data type that GenMapp supports is gene expression experiment data. GenMapp does not allow a user to dynamically select genes of interest to be overlaid or allow for operations to be performed on the data from within the diagrams (such as searching/filtering/sorting data from within the pathway diagrams).

A published case study titled "Visualising gene expression in its metabolic context" (Wolf et al., Briefings in Bioinformatics. Vol 1. No 3. September 2000) discusses a visualization tool for visualizing both protein abundance and mRNA expression data in the context of metabolic pathway diagrams. The mRNA expression data was derived from Affymetrix microarrays, and the pathway diagrams were constructed in-house or imported from KEGG (Kyoto Encyclopedia of Genes and Genomes, http://www.genome.ad.jp/kegg/kegg2.html). However, this system only supports overlaying of protein and mRNA expression data, but not other data types. Further, this system does not allow dynamic data selection or performing operations on the data from the diagram (such as searching/fittering/sorting data from the pathway diagrams).

The Rosetta Resolver System (http://www.rosettabio.com/products/resolver), Cognia Pathway Builder (http://www.cognia.com), GeneSpring, by Silicon Genetics (http://www.silicongenetics.com), and Cytoscape (http://www.cytoscape.org) each allow mapping of experimental data onto pathway diagrams constructed by the particular software, but the types of data supported are limited, and data cannot be overlaid onto standard, existing pathway diagrams. Multiple data sets cannot be overlaid, dynamic data-selection is not supported, and performing operations on the data from the diagram (such as searching/filtering/sorting data from the pathway diagrams) is not supported/allowed.

GeneAround (http://db.aist-nara.ac.jp/genearound/) is a software tool designed to visually explore GO (Gene Ontology) hierarchy. No integration with experimental data is supported.

TreeMap Cluster View, from Duke University (http://www.dbsr.duke.edu/software/applications/treemapclusterview/default.htm) allows for simultaneous navigation between a tree view and microarray data. Some integration with GO hierarchy is also be supported.

MappFinder, from the UCSF Conklin Lab (http://genomebiology.com/content/pdf/gb-2003-4-1-r7.pdf integrates a tree browser for the Gene Ontology (GO) Project with GenMAPP. This allows an interactive browser that can identify those GO terms from a user-supplied list that have an over-represented number of gene expression changes.

E-Cell (http://ecell.sourceforge.net/) is a research project aimed at developing necessary theoretical supports, technologies and software platforms to allow precise whole cell simulation.

U.S. Patent No. 6,496,832 to Chi et al. discloses a system for analyzing data organized into data sets and for transforming the data sets into a visual, spreadsheet representation of the same. No applications to representations of biological data or interactive manipulation of such data are disclosed.

As noted above, although various attempts have been proposed, all the proposed techniques have limited functionality, not the least of which is that the representations are not standard, and/or do not allow for user annotations, control over data filtering, or editing of standard diagrams. More powerful tools are needed for additional manipulation of data associated with biological/biochemical charts and diagrams that will enable users to more easily and more effectively make use of the enormous amounts of data that are relevant to such charts and diagrams.

### SUMMARY OF THE INVENTION

The present invention provides systems, methods and computer readable media for visualizing biological data by displaying an interactive biological diagram created by any one or combination of the following: importing pre-existing static graphical images; importing graph data structures from pre-existing databases; importing pre-existing local format objects; semi-automatically or automatically extracting from text; or manually constructing the diagram. Furthermore, such biological diagrams may be linked to other sources of biological knowledge, such as scientific literature, experimental data sets, other biological diagrams, etc. Furthermore such linking may be done via local format.

The linked biological concepts/relationships, so linked from the at least one other data source/database, may be mapped on the biological diagram in a location from which the linked semantics were extracted, such as by overlaying graphical representations of the concepts/relationships for example. The overlaid biological diagram is displayed and can be interactively used by a researcher.

The present invention provides systems, methods and recordable media for creating, modifying and extending a collection of items that embodies the interests of the user, and is referred to as the user context. The user context may include a set or sets of concept(s), relationship(s), entity(ies) and/or interaction(s) that a user is interested in researching. The user context may be represented in a local format.

The present invention provides systems, methods and computer readable media for managing user context. User context items may be presented to the user in a graphical or other interface. Through such an interface, user context items may be viewed, created, modified, deleted and/or extended.

The user context may be generated manually by a user via selecting and inputting such items into the user context. Modification and extensions of the user context may also be performed manually.

Additionally or alternatively, creation, modification and/or extension of the user context may be carried out by operations initiated from within an interactive biological diagram. For example, user context may be generated from the information selected from the diagram or portion thereof. [for example, the user could do a right mouse click on a diagrammatic element and select the 'option: add to user context' to add it to user context]

Further, the present invention provides for creation, modification and/or extension of the user context from operations initiated within other data sources, such as from text viewers or experimental data viewers.

Further, the present invention provides systems, methods and computer readable media for identifying a set of user context items in one or more biological diagrams. This functionality is useful for overlaying user context items onto one or more biological diagrams. This functionality is also useful for searching, filtering and organizing a corpus of biological diagrams and linked data sources based upon the number of items from a selected set from user context items, that the biological diagrams contain.

Annotations may be attached to one or more biological diagrams, or to one or more diagrammatic elements contained within one or more diagrams by the present invention. Such annotations may take many forms, including, but not limited to, freehand drawings, text, images, links to data, or data. Such annotations may be overlaid for viewing them on the one or more biological diagrams. Further, one or more diagrams may be accessed to select one or more concepts and/or relationships for viewing the annotations associated with the selected concept(s)/relationship(s).

Furthermore, such annotations may be used to search, filter, organize or overlay one or more diagrams or linked data sources.

The invention further provides systems, methods and computer readable media for displaying comparing two or more biological diagrams. Similarities and discrepancies of information represented in these diagrams can be displayed on the respective diagrams. For example, properties of each diagram that are in agreement, and/or properties that conflict may be highlighted, annotated or otherwise brought to a user's attention. Further, a single new biological diagram may be generated representing the combined information (such as similarities and/or discrepancies) in the above-mentioned biological diagrams.

The invention further provides systems, methods and computer readable media for displaying two or more interactive biological diagrams. Functional interaction is provided among the various views. Operations that can be performed in biological diagrams include selecting concepts or relationships (entities and interactions), or subgraphs. Such operations initiated in a biological diagram may initiate responses in other biological diagrams. For example, a concept or relationship displayed on a biological diagram may be selected to not only locate that concept/relationship in one or more or more of other biological diagrams, but to automatically initiate an operation [such as -see later claims, but includes searching, filtering, etc] in the one or more of the other biological diagrams with respect to that data, based on the selected concept/relationship.

The invention further provides systems, methods and computer readable media for displaying one or more interactive biological diagrams and one or more displays of contents from at least one database, such as an experimental database or textual database, for example. Functional interaction is provided among the various views. Operations that can be performed in biological diagrams include selecting concepts or relationships (entities and interactions), or subgraphs. Such operations initiated in a biological diagram may trigger responses in the said displays of contents from at least one database, such as an experimental database or textual database, for example. For example, a concept or relationship displayed on a biological diagram may be selected to not only locate that concept/relationship in one or more linked databases, but to automatically initiate an operation [such as - see later claims, but includes searching, filtering, etc] in the one or more linked data bases [such as text, experimental data etc.] with respect to that data, based on the selected concept/relationship.

The invention further provides systems, methods and computer readable media for displaying one or more interactive biological diagrams and one or more displays of contents from at least one database, such as an experimental database or textual database, for example. Operations can be performed in said displays. Such operations initiated in the said display may trigger responses in the biological diagrams. For example, a concept or relationship displayed on the said display may be selected to not only locate that concept/relationship in one or more said biological diagrams, but to automatically initiate an operation [such as -see later claims, but includes searching, filtering, etc] in the one or more said biological diagrams with respect to that data, based on the selected concept/relationship.

The invention further provides systems, methods and computer readable media for performing operations (such as overlaying, searching, filtering, and/or organizing) on one or more biological diagrams and/or displays of contents from at least one database, such as an experimental database or textual database, for example based on one or more of a diagrammatic motif; element or set of elements (e.g., entity(ies), interaction(s), concept(s) and/or relationship(s)); subgraph (i.e., portion of a diagram); or user context.

The present invention provides systems, methods and computer readable media for converting a pre-existing static graphical image into an interactive biological diagram, by extracting semantics from the pre-existing static graphical image in one or more areas where the pre-existing static graphical image displays biological semantic meaning; and linking at least one biological concept, from at least one database (of diagrams, experimental data, textual data or other representations of biological knowledge) previously unconnected with the pre-existing static graphical image, with semantics extracted from at least one region on the pre-existing static graphical image to which the at least one biological concept relate. Furthermore, such linking may be done via local format.

Still further, the present invention provides systems, methods and recordable media for displaying multiple biological diagrams in a single display in an ordered manner. An example of such an ordering would be a tabular arrangement, creating a spreadsheet-like viewer where the individual cells each contain a biological diagram. Such a collection of diagrams displayed in an ordered manner can function as a diagram itself. Further, present invention also allows the ability to view a particular cell in its own display window in order to view the biological diagram that it contains in greater detail.

Further, the multiple diagrams may be functionally linked with one or more databases for simultaneously functioning therewith. By displaying information in such an arrangement, the present invention is able to easily handle the overlay of particular types of data. For example, given a data set with multiple values that vary according to some property such as time or spatial location each set of data could be displayed in adjacent cells in a "filmstrip-like" fashion. Examples of ways in which to arrange cells include but are not limited to time sequence, biological location, experimental protocol, or user selected ordering. Additionally, by displaying multiple diagrams simultaneously in a single display, interactions and relationships with participants found in different biological diagrams can be identified, visualized and/or overlaid.

The present invention provides systems, methods and computer readable media for applying known algorithms and techniques for traversing and computing across graphical and network data structures to the biological diagrams which are represented by such graphical and network data structures. Examples of applications include, but are not limited to, determining the shortest path between a pair of nodes in a network, determining a spanning tree for a given node, computing all available pathways that connect a pair of nodes in a graph, identification of subgraph or motif structures within a graph, and the like.

The present invention further covers forwarding a result obtained from any and all of the methods and techniques described herein, to a remote location; transmitting data representing a result obtained from any and all of the methods and techniques described herein, to a remote location; and/or receiving a result obtained from any and all of the methods and techniques described herein, from a remote location.

These and other advantages and features of the invention will become apparent to those persons skilled in the art upon reading the details of the present invention as more fully described below.

### BRIEF DESCRIPTION OF THE DRAWINGS

Fig. 1 shows a schematic representation of an example architecture provided for converting static images of biological models to editable/modifiable biological models constructed in a local format.

Figs. 2A-2B show schematic representations of an example of use of an existing biological diagram to overlay experimental data on the same and identify the experimental entities/relationships on the biological diagram.

Fig. 3 shows a generalized procedure for overlaying data on a biological diagram in accordance with the present invention.

Figs. 4A, 4B, 5A and 5B show examples where experimental data is displayed in an experimental data viewer, in association with entities from the experimental data being mapped on a biological diagram.

Figs. 6A-6B show another schematic representation of an example in which the present system overlays data on an existing biological diagram.

Figs. 7A-7B show an example of producing overlays to visualize information/data from a corpus/collection of scientific text documents on top of an existing biological diagram.

Fig. 8 is a schematic representation of a tabular or spreadsheet-like viewer according to the present invention.

Fig. 9 is a block diagram illustrating a typical computer system which may be employed in carrying out the present invention.

### DETAILED DESCRIPTION OF THE INVENTION

Before the present methods, systems and recordable media are described, it is to be understood that this invention is not limited to particular method steps, hardware or software described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "an", and "the" include plural referents unless the context clearly dictates otherwise. Thus, for example, reference to "an overlay" includes a plurality of such overlays and reference to "the article" includes reference to one or more articles and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEFINITIONS

In the present application, unless a contrary intention appears, the following terms refer to the indicated characteristics.

The term "biological diagram", as used herein, refers to any graphical image, stored in any type of format (e.g., GIF, JPG, TIFF, BMP, diagrams on paper or other physical format, etc.) which contains depictions of concepts found in biology. Biological diagrams include, but are not limited to, pathway diagrams, cellular networks, signal transduction pathways, regulatory pathways, metabolic pathways, protein-protein interactions, interactions between molecules, compounds, or drugs, and the like.

A "biological concept" refers to any concept from the biological domain that can be described as one or more "nouns" according to the techniques described herein.

A "relationship" or "relation" refers to any concept which can link or "relate" at least two biological concepts together. A relationship may include multiple nouns and verbs.

An "entity" or "item" is defined herein as a subject of interest that a researcher is endeavoring to learn more about, and may also be referred to as a biological concept, as belonging to that larger set. For example, an entity or item may be one or more genes, proteins, molecules, ligands, diseases, drugs or other compounds, textual or other semantic description of the foregoing, or combinations of any or all of the foregoing, but is not limited to these specific examples.

An "interaction" relates at least two entities or items. Interactions may be considered a subset of "relationships".

An "annotation" is a comment, link, or metadata about an object, entity, item, interaction, concept, relationship, diagram or a collection of these. An annotation may optionally include information about an author who created or modified the annotation, as well as timestamp information about when that creation or modification occurred.

The term "user context" refers to a collection of one or more objects, entities, items, interactions, concepts and/or relationships that describe the interests of a user when operating the present system. User context may include a set or sets of concepts and relationships.

A "database" refers to a collection of data arranged for ease and speed of search and retrieval. This term refers to an electronic database system (such as an Oracle database) that would typically be described in computer science literature. Further this term refers to other sources of biological knowledge including textual documents, biological diagrams, experimental results, handwritten notes or drawings, or a collection of these.

A "biopolymer" is a polymer of one or more types of repeating units. Biopolymers are typically found in biological systems and particularly include polysaccharides (such as carbohydrates), and peptides (which term is used to include polypeptides and proteins) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another.

A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.. For example, a "biopolymer" includes DNA (including cDNA), RNA, oligonucleotides, and PNA (peptide nucleic acid) and other polynucleotides, regardless of the source. An "oligonucleotide" generally refers to a nucleotide multimer of about 10 to 100 nucleotides in length, while a "polynucleotide" includes a nucleotide multimer having any number of nucleotides. A "biomonomer" references a single unit, which can be linked with the same or other biomonomers to form a biopolymer (for example, a single amino acid or nucleotide with two linking groups one or both of which may have removable protecting groups).

An "array" or "microarray", unless a contrary intention appears, includes any one-, two- or three-dimensional arrangement of addressable regions bearing a particular chemical moiety or moieties (for example, biopolymers such as polynucleotide sequences) associated with that region. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other). An "array layout" refers to one or more characteristics of the features, such as feature positioning on the substrate, one or more feature dimensions, and an indication of a moiety at a given location. "Hybridizing" and "binding", with respect to polynucleotides, are used interchangeably. A "pulse jet" is a device which can dispense drops in the formation of an array. Pulse jets operate by delivering a pulse of pressure to liquid adjacent an outlet or orifice such that a drop will be dispensed therefrom (for example, by a piezoelectric or thermoelectric element positioned in a same chamber as the orifice).

When one item is indicated as being "remote" from another, this is referenced that the two items are at least in different labs, offices or buildings, and may be at least one mile, ten miles, or at least one hundred miles apart.

"Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

A "processor" references any hardware and/or software combination which will perform the functions required of it. For example, any processor herein may be a programmable digital microprocessor such as available in the form of a mainframe, server, or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic or optical disk may carry the programming, and can be read by a suitable disk reader communicating with each processor at its corresponding station.

"May" means optionally.

Methods recited herein may be carried out in any order of the recited events which is logically possible, as well as the recited order of events.

A "node" as used herein, refers to an entity, which also may be referred to as a "noun" (in a local format, for example). Thus, when data is converted to a local format according to the present invention, nodes are selected as the "nouns" for the local format to build a grammar, language or Boolean logic.

A "link" as used herein, refers to a relationship or action that occurs between entities or nodes (nouns) and may also be referred to as a "verb" (in a local format, for example). Verbs are identified for use in the local format to construct a grammar, language or Boolean logic. Examples of verbs, but not limited to these, include upregulation, downregulation, inhibition, promotion, bind, cleave and status of genes, protein-protein interactions, drug actions and reactions, etc.

The term "local format" or "local formatting" refers to a common format into which knowledge extracted from textual documents, biological data and biological diagrams can all be converted so that the knowledge can be interchangeably used in any and all of the types of sources mentioned. The local format may be a computing language, grammar or Boolean representation of the information which can capture the ways in which the information in the three categories are represented.

Biological diagrams are great repositories for information related to the current understanding of the mechanisms underlying various biological processes. Given the tremendous amounts of data being generated by current high-throughput technologies in the life sciences, there is a need for researchers to be able to identify information about entities of interest from existing biological diagrams, and be able to verify/validate these using proprietary experimental results in an efficient, computationally-assisted manner. Although a number of biological diagram databases have been developed (both public domain and proprietary) that allow users to query and download biological diagrams of interest, once downloaded, they are very difficult for the user to work with. Although they can be readily viewed, the tools for editing and extending such diagrams, through either graphical annotations or graphical overlays, based on new knowledge and data, are extremely limited, as noted above. Further, annotation of existing diagrams is not supported. Often the user has a very great amount of experimental data that needs to be analyzed/compared, and manual comparison of such data with one or more models is extremely tedious to the point that it is effectively impractical to do with any amount of efficiency.

Biological diagrams may be dependent upon or relate to many different cellular processes, genes, and various expressions of genes with resultant variations in protein and metabolic abundance. Correlation and testing of data against these diagrams is becoming progressively more tedious and time-consuming, given the increasing efficiencies in the abilities and speeds of high-throughput technologies for generating gene expression, protein expression, and other data (e.g., microarrays, RT-PCR, mass spectroscopy, 2-D gels, etc.), and with the consequent increasing complexity and number of diagrams that describe this data. Additionally, there are many sources of textual information that describe or relate to the concepts and relationships depicted in biological diagrams. Organization and referencing of these textual materials with related items in biological diagrams has become an organizational nightmare.

The present invention provides systems, methods and recordable media for mapping biological concepts and relationships to regions, on graphical images that have biological semantic meaning, where those concepts and relationships are located. Once this mapping is accomplished, the biological diagram can be used as a very powerful tool for a researcher. For example this application could take a known standard biological diagram and visually superimpose (overlay) data/information (including information in user context, for example) as well as annotations or further extensions to the diagram itself based on new knowledge and/or data, on top of it. Such superimposition allows researchers to examine their data of interest in the form that they prefer (e.g., native data format, text format or graphical format) in the context of previously defined knowledge which is represented by the diagram. Moreover, such an overlay can allow for easy understanding of data with respect to a static model represented by the diagram.

In addition to allowing for a number of different data types to be overlaid onto the diagram, the present invention also allows for a number of visualization techniques to be utilized for these overlays. Overlays may be in the form of polygons, color-coded or not, highlighting, heat map-style encoding, exaggeration, sizing, stipple patterns, texture, animation, layering, slider bars and/or other technique for drawing attention to the matched locations. A particular overlay may represent many pieces of information including, but not limited to: quantitative values, Boolean values or "presence of" data, or even graphics representing a more detained. More contextual or more expansive biological representation, whether hand-drawn or drawn using alternative types of software to that presently used in making overlays, in a semi- or fully automated fashion.

Furthermore, the present invention allows user manipulations of graphical elements within the biological diagram (that have semantic meaning) to cause operations on that data in other viewers or programs (such as text or experimental). Support for dynamic data selection coupled with data manipulation initiated from within the diagram allows for round-trip interactions between the diagram viewer and any other data viewer, for use as an exploratory data analysis application.

The present invention is applicable to use with many different types of data and/or multiple data types, as well as combinations of multiple data types, including, but not limited to protein presence data, protein abundance data, protein data in conjunction with gene-expression data, metabolite concentration data, presence of user context items, and/or frequency of occurrence within a corpus of text documents.

In addition to the many different types of data supported by the present invention, as indicated above, the present invention also supports multiple sets of data, either of homogeneous or heterogeneous types, to be overlaid onto a single biological diagram, or a collection of such diagrams, simultaneously.

Further, the present invention supports multiple sets of data, either of homogeneous or heterogeneous types, to be overlaid onto a single biological diagram, or a collection of such diagrams in an animated or propagated fashion. This technique may be used, for example, to show the output of a simulation tool at each step of the simulation. Further, this technique is useful for time-series data or data that varies according to some other singular experimental variable such as spatial location, for example.

Dynamic data selection is also supported. This is an interactive feature of the present invention which enables a user to select a subset of items in the data file(s) to be overlaid, and change this selection dynamically. A change in the selection automatically updates the information in the diagram viewer in real-time.

Creation, modification and extension of user context from operations within a biological diagram are also supported. A group of elements selected from within a diagram may be used to create a set of user context items corresponding to that group of items in the diagram.

Creation, modification and extension of user context from operations within an experimental or textual viewer is also supported by the present invention. A group of elements selected from within an experimental or textual viewer may be used to create a set of user context items corresponding to that group of items in the diagram. For example, a user may select an abstract, textual document, or a portion thereof and import it into textual viewer as described in Application Serial No. (Application Serial No. not yet assigned, Attorney's Docket No. 10030986-1). All entities and interactions in the textual document (or portion of a document) based on a predefined user context may be identified using the text viewer. The user context includes, for example, a list of keywords. Each entry in the user context generally includes an identifier as to whether the entry is a noun or a verb; the name of the entry (i.e., which contributes to the lexicon for searching); the type that the entry is (e.g., cell, process, disease, or the like for nouns; bind, promote, inhibit, or the 1 ike for verbs); and aliases for the name of the entry, which are also added to the lexicon. However, the user context may still function with only a subset of such information, although less effectively (e.g., aliases could be omitted for some entries). Of course further descriptive information categories could be included for characterizing one or more entries in the user context, as would be readily apparent to one of ordinary skill in the art.

Additionally or alternatively, an existing diagram (whether manually drawn or a pre-existing machine format diagram) or portion thereof may be used to define a user context. Using diagram viewer 400, for example, the diagram or portion thereof is converted to the local format. Once the conversion has been completed, the local format representation of the nouns and verbs represented diagrammatically are populated into the user context upon which a textual analysis may be based. Alias management of these nouns and verbs is also performed. More generally, any information which has been converted to the local format (e.g., experimental data, or other data) may be used to populate the user context.

The present invention allows a set of user context items to be overlaid onto a biological diagram or set of diagrams, according to the previously mentioned overlay techniques. For example, the "presence of' an item from a set of user context items may be overlaid onto a biological diagram or set of diagrams. Further, dynamic selection of such sets or user context items is also supported.

A corpus of biological diagrams may be searched, filtered and/or organized based upon the relationship of each diagram to a predefined set of user context items. Such a relationship might simply be the presence of one or more user context items within the diagram. However, more complex relationship criteria may be used, such as a Boolean expression describing a combination of presence and absence of identified items in a set of user context items, for example.

The present invention provides for the use of a biological diagram as a navigation tool within a corpus of data relating to the information contained in the biological diagram. In this way, the biological diagram functions as a visual table of contents of the corpus of data. Links may be provided to map experimental, textual or other data from the corpus of data to the biological diagram thus allowing easy navigability to and from the diagram and user data that is contained in textual documents, experimental databases and other sources of biological information.

Further, data may be manipulated by operations performed on elements (items) of a biological diagram. A diagram viewer according to the present invention may provide one or more mechanisms for performing multiple operations on different types of data (textual, experimental, reference data, etc.), such as searching, filtering, sorting, consistency checking, or some other computational analysis from within the diagram.

Multiple graphical biological representations are also supported by the present invention. The flexible design of the present system allows it to be used to import any graphical image, provided that a mapping can be established between biological concepts and/or relationships contained in the graphic and the coordinates where those concepts/relationships are located within the graphic.

The present system and methods automate name mapping, location searching and color overlaying of data, and give users prompt and reliable results, as well as extended capabilities, such as the ability to add diagrammatic elements, annotations, and other types of information. Furthermore, the results of any particular overlay may be stored electronically and can therefore be persisted in a format which can be saved and interpreted by other software programs for later use by the user or shared with other colleagues. High flexibility is provided to users by the present invention with regard to user control and dynamic data selection. Users may interactively select one or more subsets of items in one or more data files to be overlaid on one or more biological diagrams. The active selection may be changed dynamically, with concurrent changes in resultant views resulting from the dynamic changes.

Data may be displayed in the context of the biological semantics represented by the spatial layout of a diagram: The present invention may use a referential biological diagram to display multiple data generated and selected by the user. Since all types of data may be displayed over a single referential diagram, the present invention allows for the data to be viewed in the context of the underlying biological semantics represented by the spatial organization of the diagram.

Annotations may be added to biological diagrams or diagrammatic elements contained within these diagrams. The annotations may optionally be overlaid on the diagrammatic elements to which they refer. Selection of a diagrammatic element provides a means for accessing and viewing the annotations pertaining to that element. Many types of information may be contained in annotations according to the present invention, including, but not limited to, freehand sketches, timestamp information, author information, textual data, image data, experimental data, URL references, and other links to other documents, biological diagrams, files, etc.

Annotations may also be used as a means for accessing biological diagrams. For example, a user may issue a query requesting the system to search, filter or organize a corpus of biological diagrams for the presence of an element containing an annotation with specific properties. For instance, the user may query the system to retrieve all diagrams with an annotation created by a user named "John" that occurred "after December 21, 2000". Additionally, or alternatively, queries may be formulated based upon other properties of annotations.

Reconciliation of user data against biological semantics represented by a biological diagram may be performed using the present invention. The present invention allows for relationships contained within a biological diagram to be superimposed (either directly or in a transformed fashion, e.g., such as by overlaying a value that is computed from some datum, by running a mathematical or logical expression over the entities/concepts in a relationship) onto experimental or textual views of data. This allows the biological semantics contained in the diagram to be used as a knowledge source, against which data values can be verified or reconciled.

Further, the present invention facilitates superimposition of relationships. contained within a diagram, onto another diagram, which may be optionally viewed simultaneously with the first diagram. The superimposition may be a direct superimposition of one or more items, or a transformation of the information may be first carried out before superimposing. For example, a value may be computed from data represented by an item or group of items, such as by running a mathematical or logical expression over entities/concepts in a relationship, and then the computed value may be superimposed on the second diagram. These features allow the biological semantics contained within one or more diagrams to be compared with those in another diagram. Further, these features allow a user to search for discrepancies and/or similarities among data and values displayed by multiple biological diagrams. For example, two or more diagrams may be compared to determine which concepts and relationships are contained within one diagram and not in other diagrams that the user has access to. As another example, two or more diagrams may be compared to search for instances where two concepts are connect by differing relationships, when comparing one diagram to another. These are only two examples of uses of the described functionality, which is very powerful and flexible in forming many variations of searches and comparisons that a user may wish to perform.

The present invention provides the capability to use known algorithms and techniques for traversing and computing across graphical and network data structures to be applied to biological diagrams which are represented with such graphical and network data structures. The application of these techniques to graphical representations of biological data can have biological significance. For example, in a graphical structure where nodes are items or entities (e.g., proteins) and the presence of an arc between two nodes signifies some relationship between the two proteins which it interconnects, then computing the spanning tree for one of these nodes results in a list of proteins in the graph which directly interact with the protein represented by the selected node. As another example, as to a graph of signal transduction wherein nodes represent proteins and links between the nodes represent signaling mechanisms that may occur between the proteins connected by the links, then computing the shortest path between two nodes in the graph suggests a possible biological signaling pathway between the two proteins represented by the two nodes. Other examples of known algorithms and techniques which may be carried out include, but are not limited to, calculation of degrees of connectedness, graph width, redundancy, redundant/alternative pathways and/or graph traversal. Additionally, searching, filtering and/or organizing a corpus of biological diagrams may be carried out based on whether the diagrams contain user-specified graphical properties. Examples of graphical properties that may be user-specified include the presence of subgraph or motif structures within a graph/diagram.

The present invention enables a user to conveniently and easily overlay biological data of multiple types (e.g., protein data such as protein presence, protein abundance, protein data in conjunction with gene expression data, metabolite concentration data, genomic data, clinical data, scientific text, etc.) generated by the user on a biological diagram (or multiple biological diagrams simultaneously), to allow examination of these data in the context of pre-compiled knowledge represented by the biological diagram(s).

Biological diagrams may be generated from a variety of input formats. The system may import graph data structures fro pre-existing databases, for example. Separate import modules may serve on a database-specific basis to allow a biological diagram to be created given information in the format of each such specific database. A collection of local format objects may be imported to the system to construct a biological diagram. Diagrams created and/or by the present system may be saved and loaded.

Another functionality provided is the ability to import static graphical images and convert them to interactive biological diagrams. For example, a system may process an image of a biological diagram and determine a mapping to the coordinates of biological concepts found in the graphic. As noted above, the present invention can process diagrams from virtually any source. Examples of such sources include, but are by no means limited to: Boehringer-Mannheim charts, Kyoto Encyclopedia of Genes and Genomes (KEGG), and directed acyclic graphs of the Gene Ontology (GO) classification scheme. The present invention may also simultaneously make use of a combination of diagrams from a single source or a combination of sources.

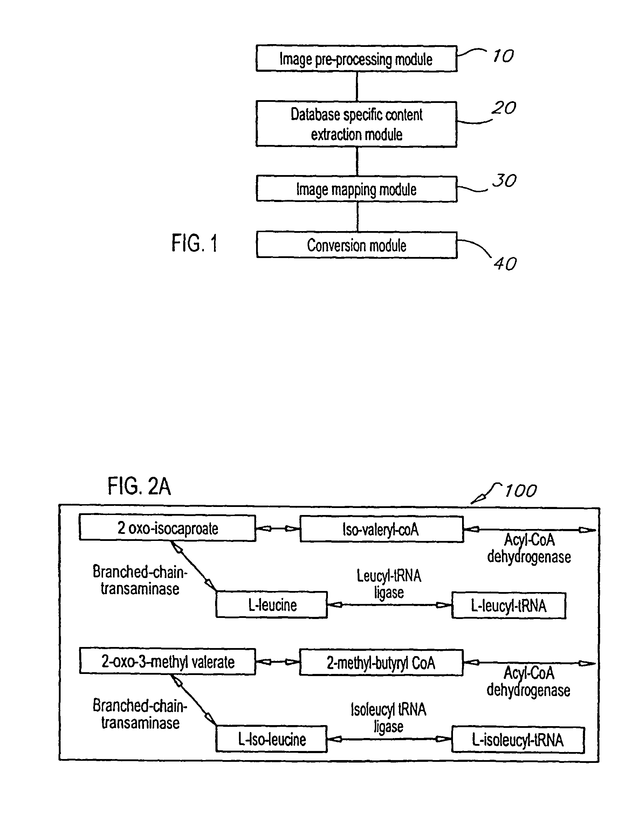

An example of processing an existing biological diagram invokes the use of an image pre-processing module 10 (see Fig. 1) which converts an image of a biological diagram into a standard format (for example, JPEG, GIF, PPM, TIFF, Bitmap), such that the image pixels in the converted image can be extracted and further processed. Images to be converted by the preprocessing module may be either digitally created or scanned from a paper source. A comprehensive list of image formats can be found in various sources, including the following web sites: http://www.dcs.ed.ac.uk/home/mxr/gfx/utils-hi.html and http://www. faqs.org/fags/grahics/fileformats-fag/parts/peamble.html. Moreover, conversion from one format to another is also very common. For example, JAVA 1.4 has an imageio library that handles interchange between a number of commonly used image formats.

Although the biological diagrams to be converted may be graphically complex and vary in format from database to database, each of the diagrams provided within the same database follow a standard/restricted format, i.e., all the symbols used to represent the various entities and relationships are standardized. For this reason, the conversion of the static images within any particular database can be facilitated with reference to the set of constraints or restrictions that are adhered to when the static image is constructed.

In this regard, a database-specific content extraction module 20 may be provided. This module stores and can access a set of constraints/restrictions which are peculiar to the database from which the biological diagram, to be converted, originated. Of course, not all biological diagrams will belong to a set of diagrams having such constraints or rules. A custom, user-constructed biological diagram may have such a set of constraints, although these constraints must have been stored in the module 20 in order to take advantage of this aspect of the processing. Thus, although this module is a valuable tool for expediting the processing of a biological diagram conversion, it is not absolutely necessary in performing an image conversion, and, in some cases, may not be available for processing a biological diagram.

In most cases involving biological repositories however, module 20 is useful and will have stored those constraints/restrictions (which are often generated by the database as a "legend" which defines these constraints) and module 20 uses these constraints to more easily identify and convert nodes and links meeting the criteria of the stored constraints/restrictions. For example, nouns may be represented as having a particular shape such as a circle or an oval. In such case, the search and identification criteria for any image processing routine is greatly focused, as circles or ovals can be readily identified as nodes. Additional criteria may even be provided, as nodes might also all be rendered in a particular color. In this way, an image identification of a blue circle, for example, would increase the confidence of identifying a node, as the process could then also be assured that the letter "O" has not been incorrectly identified as a node if all the text in this type of biological diagram happened to be in black and was identified as such in the legend.

Similarly, links may be represented as something other than simply a line or arrow connecting two or more nodes. For example, a link or reaction may also be identified by a geometrical shape, such as a rectangle or any other shape that would be used consistently throughout the diagram. Again, color may be used, alternatively, or in addition thereto. When different colors are used for links and nodes, this greatly reduces or eliminates any proximity considerations for identification of the connection of nodes by links (e.g., determining where one begins and the other ends). Further, color coding can greatly reduce or eliminate uncertainty as to whether a node has been identified, or whether it is just something that might look somewhat like a node visually. Subdivisions of nodes and links may also be separately identified by a legend (e.g., one type of node, a ligand is represented as an oval, while another type of node is represented as a circle, with or without separate colors further distinguishing the two). Or, a combination of nodes and links may be represented as a chain of reactions within the overall biological model using another identifier in a legend (shape, color, size).

Processing by the database site-specific content extraction module begins with accessing the stored constraints that relate to the particular biological diagram image that is to be converted. In the case where the image came from a database on the intemet, then the HTML address of the image has the name of the database. If the name of the database does not appear on the selected image, then the user can select the database from a list of databases, or a legend from a list of legends. Using the appropriate constraints, image processing proceeds in a much more efficient and accurate manner. For example, if a green circle is identified and the legend referred to defines nodes as green circles, a node can be automatically generated in this situation with a high degree of confidence in its accuracy. In addition to the higher confidence level, this type of conversion requires a great deal less processing capacity than a method of identification which uses approximation techniques requiring many iterations of processing just to determine where a node begins and a link ends, for example. As another example, a legend may identify a link as a yellow line. The module can then easily extract the links as they will be lines having different colors than the nodes, text or other characters in the diagram. Image processing techniques which may be performed by the database-specific content extraction module 20 include one or more of the following: color- and/or shape-processing, morphological analysis (open/close operators), connected component analysis, edge detection, detecting geometrical shapes, template matching, detecting text in the image, etc.) applied to the entire image or regions in the image (which may be pre-selected manually or automatically based on color-processing, shape-processing, and/or connected component analysis).

Another conversion aid which may be used in addition to, or alternatively to the database-specific content extraction module is an image mapping module 30. Some biological diagrams, particularly some of those which are electronically downloaded from the internet, are provided with hyperlinks at various locations on the diagram indicating areas of interest in a pathway. By clicking on one of these hyperlinks, additional information regarding that location on the pathway of the biological diagram is accessed. Such information may include location of a particular node or link, name of the entity or reaction represented by the node or link, and/or other more specific information characterizing what it is being represented at that location on the pathway. Image mapping module 30 accesses these hyperlinks and uses the additional information that is accessed to help generate a biological diagram in a local format.

Additionally, many internet sites or databases associate an image map with the static image of the biological diagram, wherein links are provided to various entities (nodes) and relations (links) represented in the image. Such information can also be used to aid the process of extraction of objects (such as identifying interesting regions in the image, etc.). Moreover, the hyperlinks and comments in the HTML image map can be used to extract further information, such as details about the genes/proteins of interest, details about various interactions, etc. Those source documents associated with a biological diagram contain the HTML language that imports and positions the image of the associated biological diagram. For example, when a page is accessed that contains the image of a biological diagram, by selecting "View" from the toolbar of a browser and then selecting "Source" from the drop down menu that ensues, the HTML language that imports and positions the image can be displayed by selecting "View" from the toolbar of a browser and then selecting "Source" from the drop down menu that ensues. Image mapping module 30 also accesses the source of the biological diagram image and extracts information regarding nodes and links to use in converting the diagram into an editable form in a local format. The HTML gives coordinates of locations on the map defining the biological diagram, so that nodes, including specific names of compounds, genes, proteins, and other species of nodes can be textually searched and matched with public databases (such as NCBI Locuslink, UMLS thesaurus, etc.) or a local database that identifies these species as nodes. Similarly, reactions, catalysts, and other varieties of "links" are identified and located by coordinates in the HTML source, which are also extracted for use in creating the local format version of the biological diagram. The information gained by the image mapping module 30 may also be used to define further restrictions for use in applying image processing and/or OCR (Optical Character Recognition) techniques by the database-specific content extraction module 20. Alternatively, the image mapping module may be configured to apply image processing and/or OCR techniques based on the information extracted.

Conversion module 40 uses the output of the database-specific content extraction module 20 and/or the image mapping module 30 and any site specific vocabulary (such as provided by a legend or other information document provided by a site or repository) to convert the view of the biological diagram into a standardized local format. OCR may be further applied to text regions of the static image to convert the text in the image into a machine readable/interpretable/editable format. Further details regarding the above-described example of processing an existing biological diagram can be found in commonly owned, co-pending Application Serial No. 10/155,675, filed May 22, 2002 and titled "System and Methods for Extracting Semantics from Images", which is incorporated herein, in its entirety, by reference thereto.

The local format used may be a computing language, grammar or Boolean representation of the information having been extracted from the static image and/or source document. As noted above, this information may be further modified or supplemented with additional information, by a user, for example. Local formatting is described in more detail in the commonly owned, co-pending application (Application Serial No. 10/154,524, filed May 22, 2002 and titled "System and Method for Extracting Pre-Existing Data from Multiple Formats and Representing Data in a Common Format for Making Overlays", and commonly owned, co-pending Application Serial No. 10/155,304, filed May 22, 2002 and titled "System, Tools and Methods to Facilitate Identification and Organization of New Information Based on Context of User's Existing Information", both of which are incorporated by reference herein, in their entireties, by reference thereto. In this way, static images from various sources and which use various criteria to represent the biological diagrams are all converted to a common local format which is also editable and modifiable by a user of the local format version.

Additionally, other sources of information relating to the biological diagrams of interest may be converted to the local format and then used for direct comparison with the information in the biological diagram, used to overlay information onto the local format of the biological diagram, and or used to supplement or modify the biological diagram. Sources of such other information include, but are not limited to, scientific text documents and experimental data. A more detailed description of converting each of these types of data into the local format, as well as processes, techniques and systems for using these various types of information (including overlaying information, inserting one form into another, converting one type to another, etc.) is given in commonly-owned, copending Application Serial Nos. 10/155,616, entitled "System and Methods for Visualizing Diverse Biological Relationships"; 10/155,675 entitled "System and Methods for Extracting Semantics from Images"; 10/155,304 entitled "System, Tools and Methods To Facilitate Identification and Organization of New Information Based on Context of User's Existing Information"; and 10/154,524 entitled "System and Method for Extracting Pre-Existing Data from Multiple Formats and Representing Data in a Common Format for Making Overlays" each of which was filed on May 22, 2002, and each of which is incorporated by reference herein, in its entirety, by reference thereto.

In this way, the present invention provides a mapping from various graphical representations (representations from multiple sources, each using its own format) to the standard local format, using techniques such as described above. Further details about such processing may be found in Application Serial Number 10/155,675 which is incorporated by reference herein, in its entirety, as noted above. The present invention is thus very flexible as to the types of diagrams that it can make use of. Any graphical image which contains biological concepts can be utilized by the present system. Hence, biological diagrams from many different sources may be processed.

Custom diagrams may also be constructed manually by a user, automatically by a computer application, or by a user being assisted by a software program according to the present invention. An example of such a software system is described in co-pending, commonly owned Application Serial No. 10/155,616. A diagram may be provided to include a canvas and a set of buttons for adding elements of a diagram being constructed on the canvas. Biological entities and their relationships may be used to construct node and link diagrams representing biological information. A pathway may be built up by dragging/dropping entities onto the diagram editor canvas when building a biological diagram. A graphical icon representing the entity appears at the drop point. There may be a set of predefined "verbs" (e.g. Inhibits, Promotes, or BindsTo) which may be used to specify relationships between "nouns".

Two "nouns" may be connected with a "verb" by selecting the "verb" from the set of buttons (optionally with associated menus) provided with the diagram editor, then drawing a line between the two graphical icons representing the "nouns." Drawing may be accomplished by selecting a source node by clicking on it, pressing down on a mouse button for a "verb", dragging the mouse sprite over to the second entity(noun), then releasing the mouse button. A color-encoded arrow appears, connecting the two graphic icons, for example a red line may be used to represent the Inhibits "verb." "Verbs" used in the diagram editor may be directional; that is, a red arrow running from a first noun to a second noun indicates that the first entity inhibits the second entity, but not the converse. Further detailed information regarding construction of custom biological diagrams can be found in Application Serial No. 10/155,616 referred to and incorporated by reference above.

Other graphical depictions for "verbs" may be used by the present invention. For example, a "verb" may be represented by a node in a diagram, within lines connecting the node representing the verb with all nodes representing entities/concepts which are related by that verb.

The present invention is flexible with regard to the types of data that can be overlaid. The nature of the biological diagram being used in a specific implementation will dictate, at least to some extent, the types of data to be overlaid on it. Existing biological diagrams depict concepts that are currently found in biology, such as genes, proteins, enzymes, and/or chemical reactions, for example. In general, if a biological concept is depicted in a biological diagram, any available information about that biological concept can be overlaid onto its graphical depiction by the systems and techniques of the present invention. Non-limiting examples of information that can be overlaid include gene expression data, protein abundance data, protein data in conjunction with gene expression data, metabolite concentration data, clinical data, and scientific text. For example, information regarding the presence of enzymes in a sample may be overlaid on the biological diagram where that enzyme is represented; the presence of proteins in a segment of scientific text may be identified by the present invention, and/or overlaid on a diagram; the presence of protein-protein interactions found in a text corpus may be overlaid, presence of metabolites found in a sample may be overlaid; and/or gene expression data may be overlaid on a biological diagram according to the present invention. As new concepts are discovered and depicted in diagrams and as new data become available for existing concepts, this present system can be extended to handle these new concepts and data types.

The data values may also originate from a variety of sources. For example, given a simulation program which calculates concentrations of molecules in a cell, such as E-cell (http://ecell.sourceforge.net/ ) the concentration values at each step may be overlaid onto a diagram. As the simulation progresses in a stepwise fashion, the values can be updated, producing an animated data overlay of the simulation process. Another example of simulation is use to represent gene expression/protein abundance levels in a network to show propagation over time and/or location.

Referring now to Figs. 2A-2B, an example of use of the present invention with existing biological diagrams is schematically shown. Examples of existing biological diagrams of this type can be found in the "Boehringer Mannheim Biochemical Pathway Chart" from Roche Applied Science (http://www.expasy.org/cgi-bin/show thumbnails.pl. The German publishing company Spektrum Akademischer Verlag owns the rights for commercial applications of the "Boehringer Mannheim Biochemical Pathway Chart", which represents a comprehensive set of metabolic pathways and cellular and molecular processes found in nature. Again, the chart displayed at (http://www.expasy.org/cgi-bin/show_thumbnails.pl), is only an example of an existing diagram that can be employed by the present invention, and the present invention is in no way limited to functioning only with this chart, but may be applied to virtually any biological diagram, as noted above. An additional Boehringer Mannheim chart, to which the procedures in the following example would apply directly, can be found at (http://www.expasy.org/cgibin/show thumbnails.p1?2). The present invention is not limited to use with only Boehringer Mannheim charts, however, as it is applicable to any other reference biological diagram with which concepts and/or relationships displayed in the diagram can be associated.

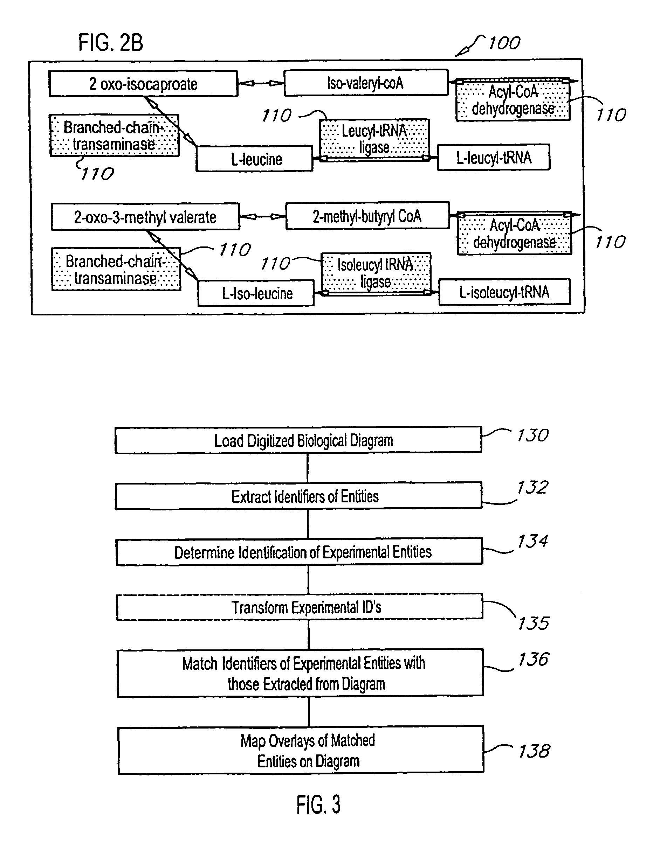

Further, only an excerpt from the Boehringer-Mannheim chart is used as a basis for the example shown in Figs. 2A-2B, in order to simplify the drawings. However, the example discussed extends to the entire chart and to other charts as well. The example shows a schematic representation 100 of panel B9 of the above-noted Boehringer Mannheim chart, (although the exact panel could be employed assuming the user is not engaging in a copyright violation) before processing according to the present invention (Fig. 2A) and after processing (Fig. 2B) to overlay 110 six enzymes with red polygons. The polygons have been drawn in a transparent red color so that the enzyme names that they cover can still be easily read. It is further noted that the choice of the color red is, in this example, arbitrary, although in other aspects of the invention, there is a semantic meaning to the color-coding scheme that is used.

The metabolic pathways chart at (http://www.expasy.org/cgi-bin/show thumbnails.pl is composed of one global panel and 120 detail panels. Image maps which contain underlying HTML documents representing the different panels (in the manner indicated above) were used to identify enzymes and their locations in these panels. The chart does not contain experimental values, but is only a map of a biological mechanism. The image maps include identifiers of the enzymes which are displayed on the chart, as well as coordinates for where each particular enzyme is mapped on the chart, with these coordinates being linked to the identifiers. The identifiers used by this particular chart are Enzyme Commission (EC numbers) which are identifiers for each particular protein(enzyme) type shown on the chart. Sometimes many different protein products will map to the same EC number which represents an enzyme type, rather than a specific molecule. In such a case, the system may provide various mechanisms for resolving ambiguity where more than one entity matches the same EC number. For example, the system may be programmed to pick the first match that it makes between the particular EC number and an entity name. Alternatively, a consolidated measure, such as an arithmetic mean, median or mode value may be calculated, if there are quantitative values to be overlaid (e.g., expression ratios), and the system then maps the candidate with the closest value to the calculated consolidated measure. Further alternatively, when quantitative values are present, it may be preferred to pick the candidate with the highest expression ratio, for example. As another alternative, a list of the entities matching any particular EC number may be displayed to the user, which the user may then select from to perform a single overlay.

There are many other identifier schemes in use in the art, including IPI (International Protein Identifier) numbers, RefSeq, and many more, as well-known to those of ordinary skill in the art. The identifiers (EC numbers) from the image map of the chart were extracted using the techniques described above (and are described further in co-pending Application Serial No. 10/155,675. As noted above a HTML image map is a graphical map of information resources accessible from a Web page. Using the image map, users can be provided with a graphical overview of any set of information resources; by clicking on different parts of the overview image, they can transparently access any of the information resources. The present invention automates this process and automatically extracts the identifiers for and locations of entities on a diagram.

The example described with regard to Figs. 2A-2B incorporated authentic proteomic data generated from a series of MS (Mass Spectrometer) experiments performed on a leukemia cell line. The experimental results from the MS were analyzed by SEQUEST (an MS interpreter software) and MASCOT (search engine for MS data) in order to identify the proteins present in the sample. While both of these products are sufficient for searching MS data, another product called Spectrum Mill, available from Agilent Technologies, Inc., Palo Alto, California, is also useful for this function, while providing still further capabilities. The output of this processing was a list of proteins indexed by their IPI (International Protein Index) and REFSEQ accession numbers. A software tool known as BNS (Biomolecular Naming System), available from Agilent Technologies, Inc., Palo Alto, California, and described in co-pending, commonly owned Application Serial No. 10/154,529, filed May 22, 2002 and titled "Biotechnology Information Naming System", was used to map the IPI and REFSEQ accession numbers available in the MS data set to their corresponding Enzyme Commission (EC) numbers. Application Serial No. 10/154,529 is incorporated herein, in its entirety, by reference thereto.

It is noted here that although the present invention used the BNS system for this example, and the BNS system is preferred for converting between various identifier systems, that the present invention is not limited to the use of the BNS system for this portion of the processing, as others schemes and software could be developed to perform the necessary conversions. The list of EC numbers corresponding to the proteins in the experimental data were then compared with the extracted EC numbers corresponding to the proteins displayed in diagram 100. For those EC numbers in the experimental data that matched extracted EC numbers, the locations of the extracted EC numbers were accessed and overlays 110 were performed over the matching proteins.

Although only six overlays 110 are shown in Fig. 2B, this is only the result for one panel, as noted above. Overall, approximately 1,600 enzymes were identified from the MS experiments (i.e. their IPI numbers matched a gene/protein symbol in LocusLink (the underlying database representing BNS). Of these, about 200 proteins were located and overlaid on the chart overall.

Fig. 3 shows a generalized procedure for overlaying data on a biological diagram in accordance with the specific example described with regard to Figs. 2A-2B above. At step 130, a digitized version of a biological diagram is loaded into the present system. At step 132, identifiers of entities displayed on the biological diagram are extracted. As noted, one way of extracting these identifiers is through the use of an image map associated with the biological diagram. At step 134, identifiers for the experimental entities in one or more experimental data sets are determined. With regard to both the biological diagram and the experimental data, it is noted that the identifiers may be determined according to one or more of a growing number of identifier schemes set up in the industry, including, but not limited to Genbank accession numbers, Unigene cluster identifiers, clone identifiers, RefSeq accession numbers, EC numbers, IPI numbers, gene symbols, Locus Identification numbers, chromosome locations, mRNA RefSeq numbers, protein RefSeq numbers, proprietary molecular identifiers, public molecular identifiers, or other identifiers.

If the identifiers associated with the experimental data are not determined according to the same identification scheme as those used with regard to the biological diagram, optional step 135 is carried out to transform the identifiers associated with the experimental data to the same identification scheme used with regard to the biological diagram. This step, when necessary, may be carried out using the BNS system, or other means of global lookup facility that is capable of translating many, if not all of the known identification schemes. Once both sets of identifiers are set according to the same identification scheme, the system then attempts, at step 136, to match the identifiers from the experimental data with those having been extracted from the biological diagram. For those entities matching, overlays are performed at step 138, on the biological diagram, in the locations where the matching entities are displayed.

The technique of overlaying allows the user to view their data in the context of the biological semantics represented in the diagram. The overlay of experimental data in this manner allows the user to identify regions of interest or to get a general broad understanding of the biological processes represented by the experimental data. For example, a specific region of a diagram may show significant presence of enzymes mapped from the experimental data overlaid thereon, indicating that the experimental variables being manipulated in the data may play some important role in the biological mechanisms represented in that region of the diagram.

After overlaying the data, the user may be able to see that the distribution of proteins found in the sample is very dense in a particular region of the diagram on which the overlays have been performed. The user may then want to focus in on this portion of their data. The present invention allows the user to filter (or selectively choose) enzymes present in a particular region or biologically meaningful network (e.g., pathway) of the diagram and view the experimental data representing those enzymes in a more detailed viewer, such as that available in the Synapsia Informatics Workbench, from Agilent Technologies, Inc., Palo Alto, California, or VistaClara, from Agilent Technologies, Inc., Palo Alto, California, which is described in detail in co-pending, commonly assigned Application Serial No 10/403,762 filed March 31, 2003, and titled "Method and System for Simultaneous Visualization And Manipulation of Multiple Data Types", which is incorporated herein, in its entirety, by reference thereto.

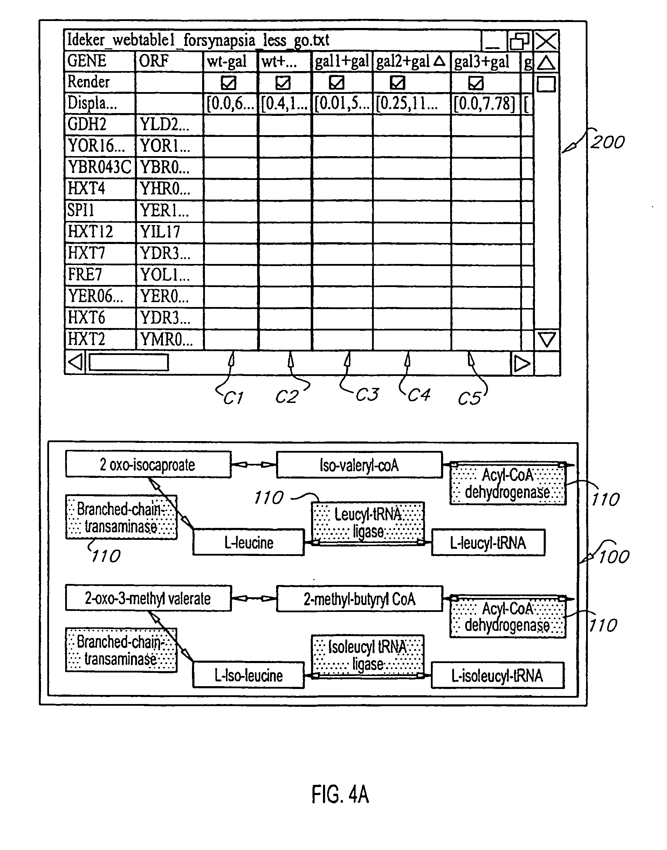

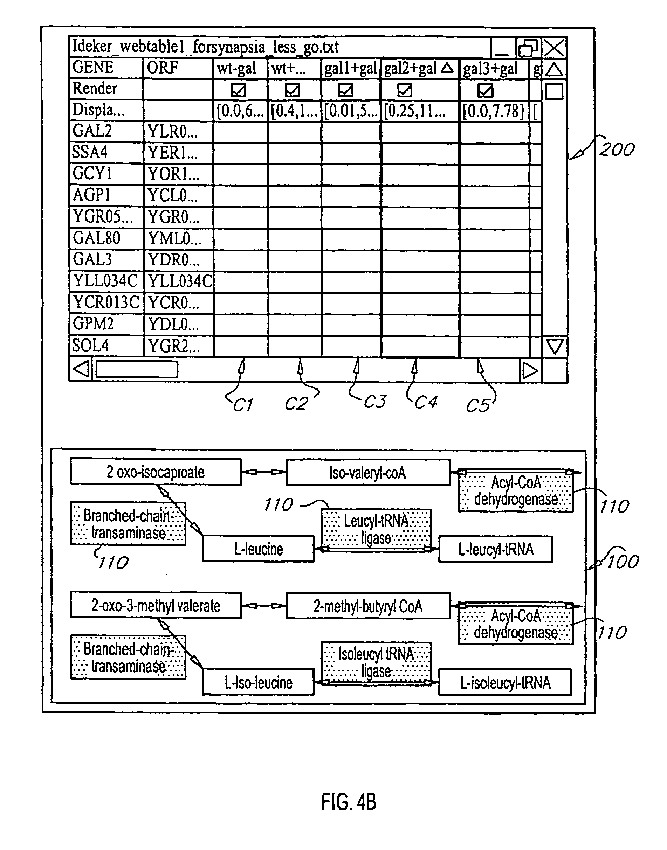

Figs. 4A-5B show examples where protein expression data is displayed in a heatmap viewer 200 (such as a Synapsia viewer, for example), in association with proteins mapped on a biological diagram 100. When a column of the heatmap is selected (such as when a user selects or "clicks on" a column) in the heatmap view 200, the corresponding expression values are overlaid onto the appropriate genes displayed in the diagram viewer 100. For example, column c2 is selected in Fig. 4A, and the overlays 110 in Fig. 4B are polygons which are color-coded to the same heat map colors as the heat map cells in view 200 for the corresponding genes. Hence the visualization technique used in this example is semi-transparent rectangles whose color matches the corresponding heatmap value for a given concept. The overlaid values are dynamically changed each time the selection in the experimental viewer changes, as noted by the variation in the colors of overlays 110 in Fig. 4B when column c4 is selected.

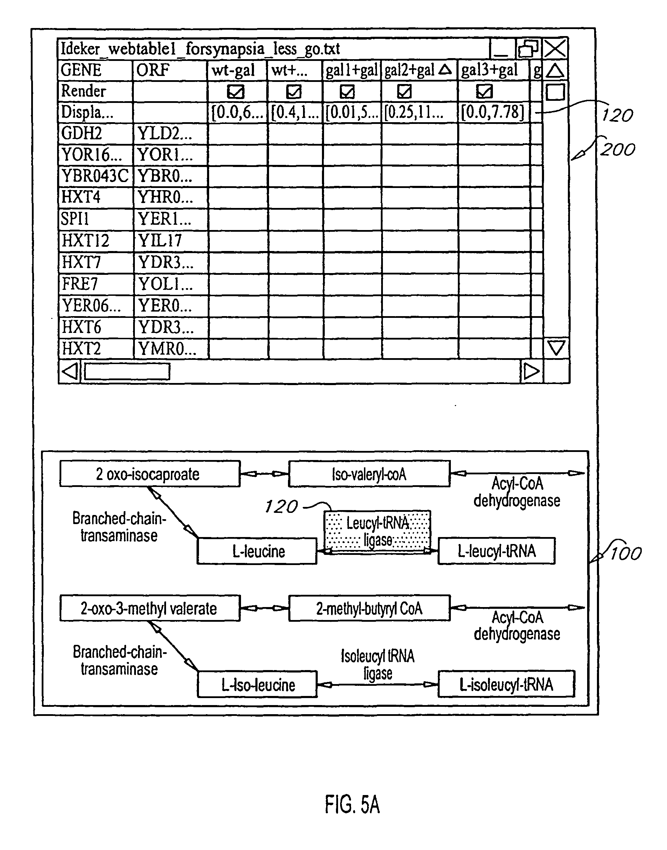

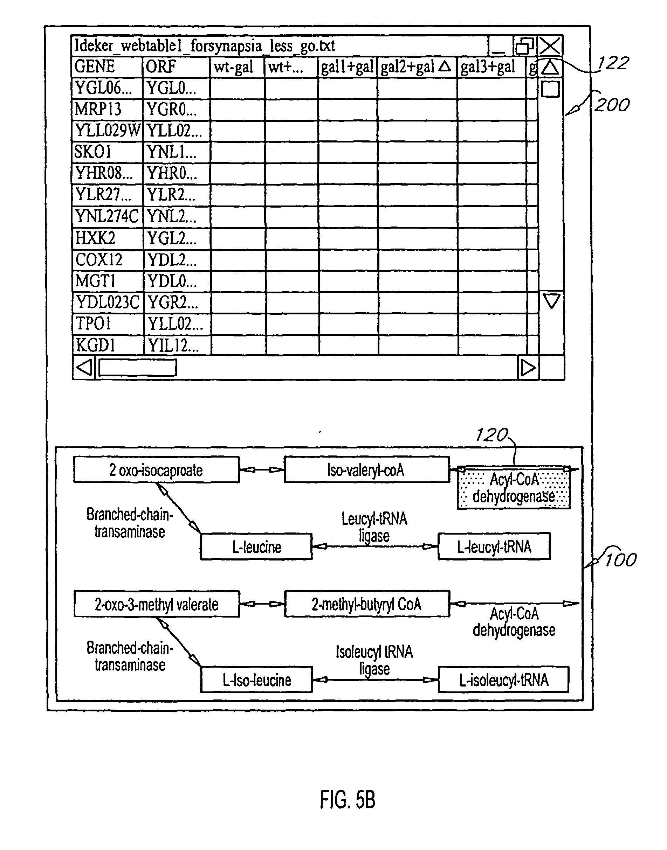

This invention also provides for user operations that are performed in the diagrammatic view 100 to cause manipulations of the view 200 of experimental data or view of scientific literature or alternative graphical views in some other window. For example, Fig. 5A shows a selection of protein 120, as indicated by the highlighted polygon in Fig. 5A. Upon selection of protein 120 in diagram viewer 100, the system automatically reorders the rows of data in view 200 so that the row of data corresponding to protein 120 is placed at the top of the experimental viewer 200. Additionally, all the other rows of data are sorted according to their similarity with that new top row 120. In this case the user operation of selection within diagram viewer 100 initiated a sort operation in experimental viewer 200 based upon the row of experimental data for the selected protein. When the user changes the selection in diagram viewer 100, such as by selecting protein 122 as shown in Fig. 5B, the rows of experimental data in viewer 200 are simultaneously reordered, with the row of experimental data corresponding to protein 122 placed at the top of the view and the other rows of data being sorted by similarity to row 122.

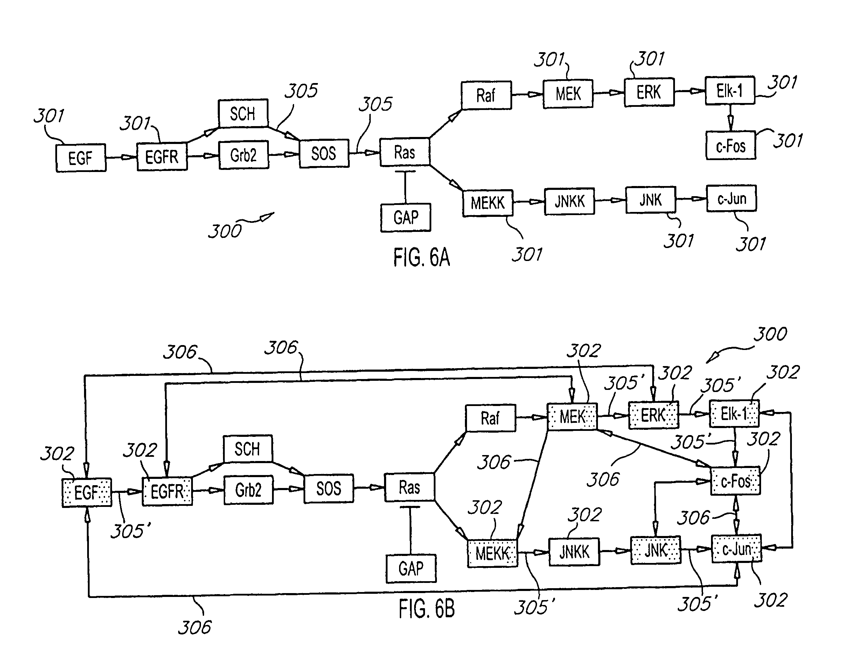

Figs. 6A-6B show another example in which the present system overlays data characterizing biological interactions on an existing biological diagram. In this example, a digitized version of the MapK signaling pathway obtained from the Kyoto Encyclopedia of Genes and Genomes (http://www.genome.ad.jp/kegg/) was used as the reference biological diagram. Fig. 6A shows a schematic representation of the MapK signaling pathway 300 with no overlays on it. Entities (in this case, proteins) are represented by non-shaded rectangles 301 and interactions between the entities are indicated by black arrows 305 interconnecting the rectangles 301 involved in the interactions depicted.

Using the image map for this diagram 300, A list of the entities depicted in this diagram was extracted using the automated methods of the present invention, and as further described in Application Serial No. 10/155,675. The list of entities was then imported to a knowledge extraction tool, which identified a corpus of scientific articles that discussed these entities. The tool used in this implementation was a software tool known as BioFerret (Agilent Technologies, Inc., Palo Alto, California) which is described in detail in co-pending, commonly owned Application Serial No. 10/033,823, filed Dec 19, 2001 and titled "Domain-Specific Knowledge-based MetaSearch System and Methods of Using". Application Serial No. 10/033,823 is incorporated herein, in its entirety, by reference thereto. However, a number of other means such as a keyword search of PubMed or other scientific database(s), for example, may be used to identify a corpus of relevant documents.

Once the corpus was identified, Bioferret was then used to search the corpus for the presence of any interactions occurring between the entities identified in diagram 300. Interactions that were identified between the entities were then overlaid onto diagram 300, as shown in Fig. 6B. Those interactions which had previously been identified in Fig. 6A are overlaid 305'in a way to alert the user that the corpus has information that coincides or agrees with the pre-existing diagram, For example, overlays 305' may be color-coded a different color than interactions 305, such as by coloring red or some other color that stands out. Although not shown, if an interaction 305' does not agree with an interaction 305 in the same location, another color coding may be used to indicate such, and/or an arrowhead in the reverse direction may be overlaid. Interactions that were newly discovered from the corpus of scientific articles (i.e., interactions not previously shown in Fig. 6A) are displayed as interactions 306 in Fig. 6B. Interactions 306 may be color-coded differently than 305 and 305', or otherwise visually distinguishable to show a user that they are newly added interactions.

Bioferret may search textual documents in the corpus, for example, to identify language that includes two of the entities (e.g., proteins or nouns) from the list that appear together with a predefined verb that indicates the presence of an interaction. Examples of the predefined verbs include, but are not limited to, upregulation, downregulation, inhibition, promotion, bind, cleave and status of genes, protein-protein interactions, drug actions and reactions. The co-occurrence of two nouns with a verb, as described, may be searched on the basis of a single sentence, paragraph, or page, for example. As noted previously, the user context may be flexibly defined to allow searching upon particular entities, interactions, concepts and relationships according to the user's interests. Further details about this logic are discussed in co-pending, commonly owned application Serial Nos. 10/154,524 and 10/155,304.

In this example, the presence of a particular interaction in a corpus of scientific text was denoted in the diagram by superimposing a red arrow 306 on the diagram connecting the two participants 302 of the interaction. Also, the rectangles representing those proteins which were participants in a reaction were highlighted by coloring the rectangle 302 representing the protein with a red color. In this way, the presence of interactions and the proteins that participate in them is overlaid onto the Kegg MapK signaling pathway diagram 300. Additionally, one or more URLs (pointers) that link back to one or more literature references from which the interaction was discovered may be overlaid on the interaction link 306 having been overlaid on diagram 300. This permits a user to click on the interaction link 306 to directly link back to the original text document or article that the interaction link 306 refers to. When more than one URL is overlaid, clicking on the interaction 306 launches a pop up menu or other selection means by which a user can select which document or article to open.

In addition to annotating existing entities on a biological diagram by overlaying as discussed above, the present invention may also perform an overlay onto the diagram to display interactions in which one of the participants is a entity 301 that existed in the original diagram 300, and the other participant was not in the original diagram. For each of these interactions, a new rectangle or other polygon is drawn on the diagram to represent the entity participant not found in the original diagram, and an arrow representing the interaction is drawn to connect the newly drawn polygon and the pre-existing rectangle 301/302. The previously unrepresented entity is an entity discovered while searching the corpus, when it is discovered that the previously unrepresented entity is described in the corpus as interacting with a previously represented entity 301.

It should be further noted here, that while various functions of the present invention are described separately herein with reference to separate figs., that these various functions are not mutually exclusive of one another. Thus, for example, in addition to overlaying the diagram of Figs. 6A-6B with information obtained from a knowledge extraction tool, the present invention can additionally overlay the same diagram with experimental data, in a manner described previously. For example, the list of entities extracted from the image map for diagram 300 (or a list of identifiers for those entities) can be compared with a list of entities (or identifiers for the same) identified in one or more experimental data files (after conversion to make sure that the same identification system is being used for both the diagram entities and experimental entities, if necessary) to identify matching entities. The entities (proteins) which result in matches may then be overlaid on the same diagram. The overlays may be performed with different annotation, such as different coloring, different shaped polygons, etc. to distinguish this overlay from the overlay derived from the information derived from the knowledge extraction tool. Still further, this diagram, with any or all of the previously described overlays, can be used in conjunction with an experimental data viewer in the manner described with reference to Figs. 4A-5B.

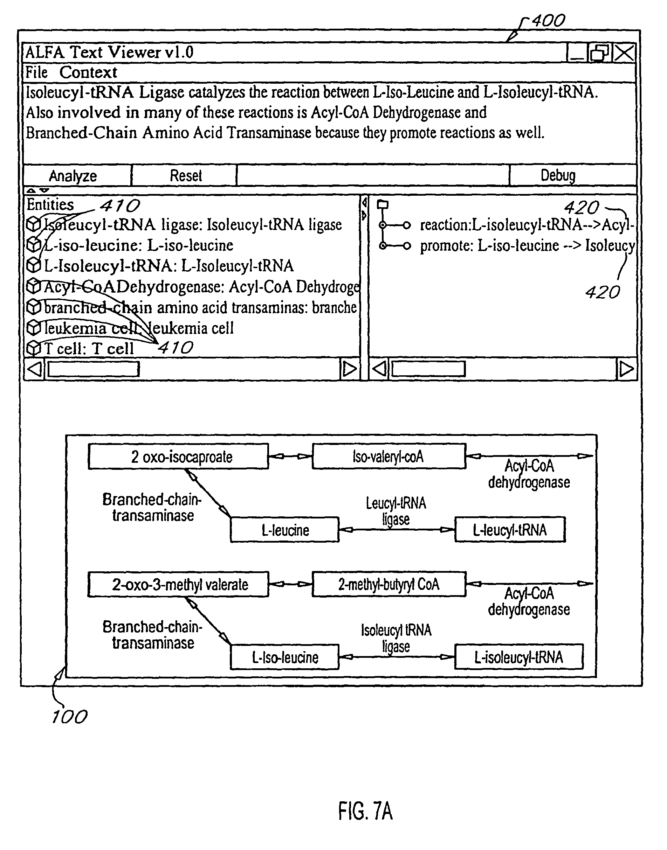

Figs. 7A-7B are referred to with regard to an aspect of the present invention for producing overlays to visualize information/data from a corpus/collection of scientific text documents on top of an existing biological diagram. In this example, the biological diagram 100 that was referred to in Fig. 2A has again been used for purposes of demonstration. Thus, the diagram 100 shown in Fig. 7A is the same as that shown in Fig. 2A, i.e., prior to any overlays. Methods and systems for user-guided knowledge extraction is described in co-pending, commonly owned Application Serial No. 10/154,524, as well as co-pending and commonly owned Application Serial No. (Serial No. not yet assigned, Attorney's Docket No. 10030986-1), titled "System, Tools and Method for Viewing Textual Documents, Extracting Knowledge Therefrom and Converting the Knowledge into Other Forms of Representation of the Knowledge" which is being filed concurrently herewith, may be used for the present aspect of the present invention. Application Serial No. (Serial No. not yet assigned, Attorney's Docket No. 10030986-1) is hereby incorporated herein, in its entirety, by reference thereto.

Using the method and system described in Application Serial No. 10/154,524 and Application Serial No. (Serial No. not yet assigned, Attorney's Docket No. 10030986-1), user-guided, semi-automated text mining techniques are used to extract "nouns" (e.g. biological entities) and "verbs" (e.g. relationships) from sentences in scientific text. The nouns searched for are the entities extracted from the image map for diagram 100 in this instance. A software program that implements this method is the ALFA (Agilent Local Format Architecture), from Agilent Technologies, Inc., Palo Alto, California, which is described in detail in Application Serial Nos. 10/154,524 and Application Serial No. (Serial No. not yet assigned, Attorney's Docket No. 10030986-1).

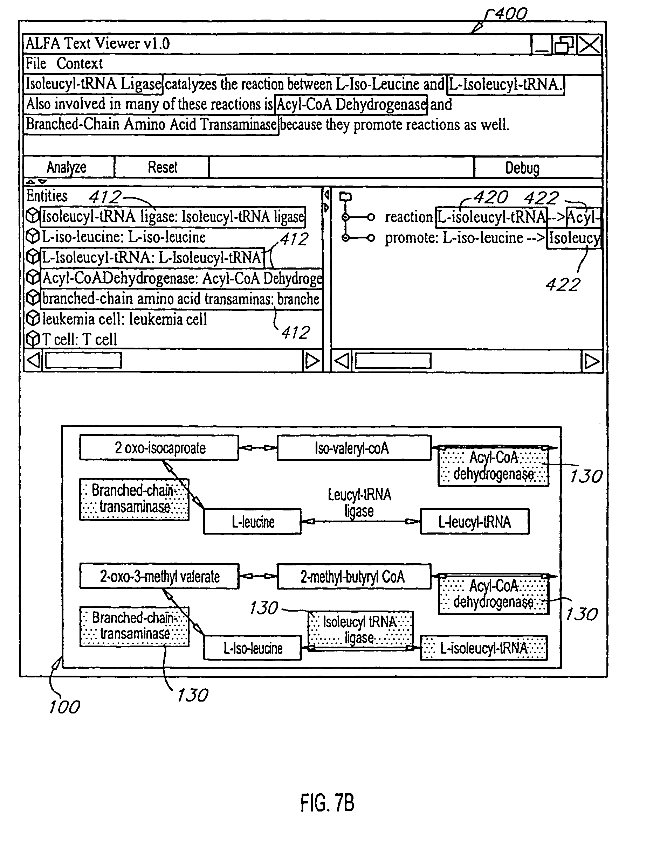

Entities from the text corpus mapping to enzymes, substrates, and products (i.e., entities) in diagram 100 are then marked/overlaid on diagram 100, as shown by reference numerals 130 in Fig. 7B. Fig. 7A shows the list of entities 410 identified in the text documents in ALFA Text Viewer 400, as well as a list of interactions 420. In Fig. 7A, no entities have been highlighted, and therefore no overlays appear on diagram 100 in Fig. 7A. When a user selects one or more entities in the text viewer 400, the selected entities are highlighted 412, as shown in Fig. 7B. Also, the interactions relating to the selected entities are automatically highlighted 422. The corresponding entities 130 in diagram 100 are simultaneously and automatically highlighted or overlaid, as shown in Fig. 7B. Thus, this aspect of the present invention provides the user with dynamic selection capabilities for customizing which entities to be overlaid on the existing biological diagram. Dynamic selection is not limited to actions performed within the ATV program. Selection could be initiated from any other application that contains a list of concepts and/or relationships found in the diagram.

The present invention further modifies the diagram 100 to be interactive, so that user operations performed in diagram 100 initiate actions that manipulate data being displayed in other programs/viewers. For example, links to the textual document(s) (and/or the specific sentences) where an entity was found in the ATV program or other text mining program may be stored in the local format object displayed in diagram 100 by the diagram viewer. In this way, when a user clicks on a marked/overlaid entity name 130 on diagram 100, all text documents that refer to entity 130 can be retrieved by the system. This results in diagram 100 showing a high-level view of enzymes, substrates, products, etc., that represent the text corpus, along with links to the relevant text, where applicable, such as in viewer 400, for example. This added functionality allows diagram 100 to operate as a "table of contents" for the text corpus, which by itself is very difficult to navigate through and to make associations for a given entity or interaction.

Further, diagram 100 can be used to perform operations on different data types pointed to by the concepts, entities, interactions and relations in the diagram. For example, the user can select a set of concepts, entities, interactions and/or relations in diagram 100 and use the selected list as the basis of a search for other documents which refer to those concepts, entities, interactions and/or relationships. An example of a software tool that may be used to perform such a search is BioFerret, which is described in Application Serial No. 10/033,823, as noted above. The diagram can thus manipulate the data being displayed in other viewers - in this case ― BioFerret, but also in other viewers, such as VistaClara, Synapsia, etc., either independently or together, simultaneously. Further, the functionality is two-way. For example, a manipulation in VistaClara can automatically perform a related manipulation in the diagram 100, etc.

Using diagram 100 as a table of contents, a user can visualize all the entities identified by the textual search on a single image 100. Using the visual patterns of marking as they appear on diagram 100, the user can select entities of interest and be linked directly to their occurrences in the text corpus. The user can then navigate to those articles that refer to the identified areas of interest. For example, all entities relating to fatty acid synthesis can be located in a specific region of the Boehringer-Manheim chart from which diagram 100 is derived. By viewing his or her data in the context of diagram 100, a user can specifically target those text documents that refer to a particular metabolic mechanism, such as fatty acid synthesis. The ability to view and navigate the data in this manner would not be possible without the superposition of data onto the semantically meaningful layout of the diagram. Moreover, the user also gets a high-level perspective of different metabolic processes mentioned in the text corpus.

Further, the user may select an entity or group of entities from diagram 100 and overlay this graphical information on experimental data (such as contained in a viewer 200, for example. Using the rules of semantics employed for generating the biological diagram 100, the system can compare what is purported by the biological diagram, with what is being shown in the experimental data. For example, if diagram 100 shows that entity A upregulates entity B, but the experimental data shows that entity A downregulated entity B during the experiment, the system would then flag the experimental data, in order to indicate its inconsistency with what is shown on the existing biological diagram. Flagging may be performed in any of the manners described above (coloring, highlighting, stippling, textual overlay, etc.)

Still further, the user may select a group of entities or concepts from diagram 100 and generate user context from them. This may be accomplished, for example, by first selecting the entities/concepts of interest with a mouse click, at which time a popup menu may appear to select conversion of the selections to a local format to be inserted into the user context. Alternatively, the user may drag and drop selections to a designated location on the screen which action then initiates conversion of the dropped items to the local format and entry into the user context. Any changes made to the user context may be displayed in real time on the screen.

Many existing biological diagrams contain depictions of small, isolated systems, as contrasted with the large overall diagrams of biochemical pathways like the Boehringer Mannheim diagrams. The KEGG diagrams are an example of diagrams of small, isolated systems (as exemplified in Figs. 6A-6B). The present invention further includes the capability of combining such biological diagrams in an ordered manner for display. An example of such an arrangement is a tabular or spreadsheet-like viewer to display a plurality of the small diagrams, in adjacent cells (e.g., thumbnails). This type of view may be generated programmatically, by inputting the individual, digitized biological diagrams into a user interface data structure in a JAVA application known as a JTable, for example.

By constructing a collage or composite of a number of smaller diagrams (e.g., tiling the display), this results in a larger biological diagram which can be subjected to the overlay, interaction and navigation techniques described above. The spreadsheet-like viewer allows information to be viewed in the context of many diagrams simultaneously, and enhances the probabilities of discovering properties among the various diagrams that otherwise, would likely would not have been noticed by viewing only a single diagram at a time. Additionally, the user can freely rearrange cells, rows and columns in the table, positioning the diagrams in ways that accentuate similarities and correlations or draw attention to disparities.

This feature also provides functionality for displaying sequential temporal views of the same biological network (e.g. in adjacent or sequential frames of the spreadsheet viewer, or to display contrasting views of how the components of a displayed network interact, based on different interpretations arrived at through different experimental methods and data, as revealed through the scientific literature and/or through pure experimental data.

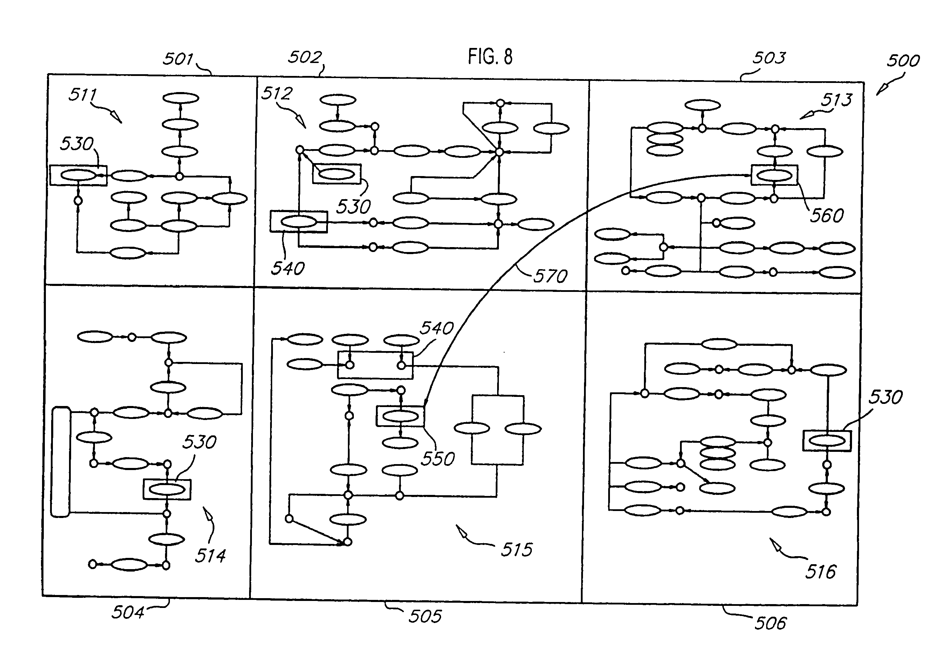

Fig. 8 is a schematic representation of a tabular or spreadsheet-like viewer 500 according to the present invention. Each pane or cell 501,502,503,504,505, and 506 contains a biological diagram 511,512,513,514,515, and 516, respectively. As noted, Fig. 8 is a schematic view. In use, it is not unlikely to display one or two dozen panes or cells in a single view. Also, the biological diagrams are, in reality, much more dense and complex than those shown. For purposes of simplicity and to meet the standards of the drawing rules imposed for patent applications (the actual biological diagrams, although clear, would appear much too small to be accepted under the rules), simplified, schematic representations of the biological diagrams and overall spreadsheet-like view have been used.

Each of the cells in Fig. 8 displays an individual biological diagram depicting a metabolic pathway, or other biologically relevant network, such as might be obtained from KEGG, or other established biological diagram database, for example. Using the techniques described above, overlays for the entities D-Glyceraldehyde-3P 530 and Pyruvate 540 have been performed. The overlay process can be applied simultaneously to all of the cells in the view 500. As can be seen, the overlay process identified the presence of D-Glyceraldehyde-3P in each of cells 501, 502, 503 and 506, while Pyruvate appears in cells 502 and 505. Although not detectable by the black and white Fig. 8, the system may differentially color the different entities for easy distinction when more than one entity is being overlaid. In this example, Pyruvate 540 overlays were highlighted/colored green and D-Glyceraldehyde-3P 530 overlays were highlighted/colored in purple. By performing such an automated overlay, a user will be able to readily identify that Pyruvate is involved in only two of the six pathways shown in the viewer 500, while D-Glyceraldehyde-3P is involved in four of the six pathways shown. By selecting one of the individual cells (using a mouse or keyboard, for example), the system zooms in on that individual cell to give a full screen detailed view of that particular cell.

Further, viewer 500 may perform overlays indicating connections between biological concepts/entities represented in the individual cells, in a manner as described above with regard to Figs. 6A-6B. In that example, however, an interaction is identified as present and therefore is overlaid only if both proteins involved in the interaction exist in the single biological diagram 300. In the present example, however, the spreadsheet-like view 500 expands this function, so that interactions whose participants (entities) each reside in two separate diagrams from separate cells can be identified. This is made possible by processing all of the image maps relating to all of the diagrams in all of the cells simultaneously. As an example of this functionality, the metabolites Acetyl CoA 550 and Acetaldehyde 560 were highlighted with blue and orange boxes, respectively. The existence of an interaction between these two compounds is indicated by a green arrow 570 that spans across cells 505 and 503 of the spreadsheet-like view 500.

It should be further noted that although two functions have been described with regard to the view in Fig. 8, that these functions are not dependent upon one another, but may be used together or separately with such a viewer. Additionally or alternatively, all of the other functions discussed above here may be fully employed with the spreadsheet-type viewer, e.g., integration with an experimental data viewer, navigating text corpus, etc.

Still further, it is noted that more than a typical dozen or two of the cells may be tiled and displayed simultaneously with viewer 500. Even if two or three hundred cells are displayed and overlaid on, it is possible to obtain meaningful, high level insight as to high density locations of entities the user may be interested in, even though the details of the entities and diagrams would not be visible until one or more cells were zoomed in on. This technique is particularly useful for temporal, locational sequences and views of different data arrived at thorough different methods with regard to the same biological network.

Another useful feature that may be provided by the present invention is to show time lapse properties of entities or relationships. This may be particularly useful for experimental data, the values of which may change over time. By performing overlays with morphing or animated GIF technology, an entity or relationship may be represented to gradually shift color, fade in or fade out, or change shape over a time sequence to indicate a changing value of that entity ore relationship during an experiment or over the course of a biochemical reaction taking place. Alternatively, variations in the entities or relationships may be displayed in a "freeze frame" fashion by tiling a time sequence of the same pathway in adjacent cells of a spreadsheet-like viewer 500.

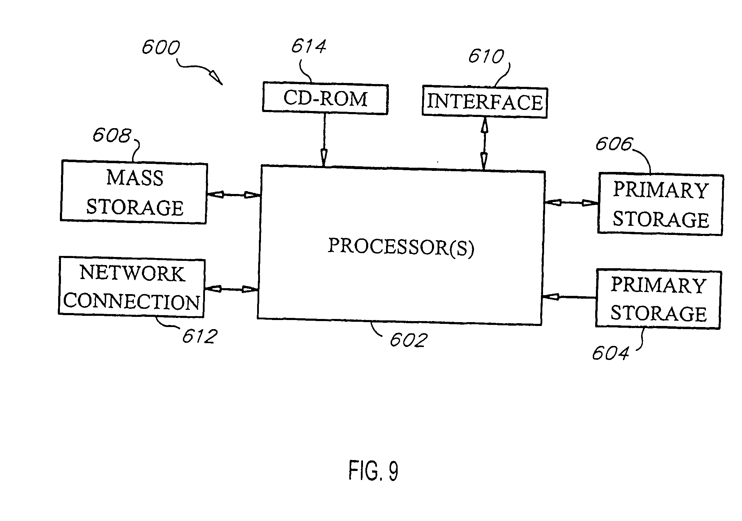

Fig. 9 illustrates a typical computer system which may be employed in carrying out the present invention. The computer system 600 may include any number of processors 602 (also referred to as central processing units, or CPUs) that are coupled to storage devices including primary storage 606 (typically a random access memory, or RAM), primary storage 604 (typically a read only memory, or ROM). As is well known in the art, primary storage 604 acts to transfer data and instructions uni-directionally to the CPU and primary storage 606 is used typically to transfer data and instructions in a bi-directional manner Both of these primary storage devices may include any suitable computer-readable media such as those described above. A mass storage device 608 is also coupled bi-directionally to CPU 602 and provides additional data storage capacity and may include any of the computer-readable media described above. Mass storage device 608 may be used to store programs, data and the like and is typically a secondary storage medium such as a hard disk that is slower than primary storage. It will be appreciated that the information retained within the mass storage device 608, may, in appropriate cases, be incorporated in standard fashion as part of primary storage 606 as virtual memory. A specific mass storage device such as a CD-ROM 614 may also pass data uni-directionally to the CPU.

CPU 602 is also coupled to an interface 610 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 602 optionally may be coupled to a computer or telecommunications network using a network connection as shown generally at 612. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for converting data types to the local format may be stored on mass storage device 608 or 614 and executed on CPU 608 in conjunction with primary memory 606, and one or more interfaces 610 (e.g., video displays) may be employed in displaying the viewer operations discussed herein.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CDRW, DVD-ROM, or DVD-RW disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, compound, process, process step or steps or order in which they are carried out, software or hardware, to the objective, spirit and scope of the present invention. All such modifications are intended to be within the scope of the claims appended hereto.

## Claims

1. A method of visualizing biological data by displaying an interactive biological diagram (100,300, 511,512,513,514,515,516), said method comprising the steps of:
providing biological information that may be displayed by a graphical representation;
converting the biological information to an interactive format in which the biological information is capable of being graphically displayed; and
assembling the converted information and visually displaying the converted information in the form of an interactive biological diagram (100,300, 511,512,513,514,515,516).

2. The method of claim 1, further comprising linking at least a portion of the interactive biological diagram (100,300, 511,512,513,514,515,516) to at least one other database or source of biological knowledge.

3. The method of claim 2, wherein said linking includes representing linked data in a local format.

4. The method of claim 1, wherein at least a portion of the biological information is provided by at least one of the steps consisting of: importing one or more pre-existing static graphical images, importing one or more graph data structures from one or more pre-existing databases, semi-automatically or automatically extracting data from text, and manually constructing.

5. The method of claim 1, further comprising annotating the biological diagram (100,300, 511,512,513,514,515,516) or a portion thereof.

6. The method of claim 5, further comprising overlaying (110) annotations resulting from said annotating on the biological diagram (100,300, 511,512,513,514,515,516).

7. A method for converting a biological diagram into an interactive research tool, said method comprising the steps of:
extracting semantics from the biological diagram in one or more areas where the biological diagram displays biological semantic meaning; and
linking at least one biological concept, entity (130,301) (130,301), interaction, or relationship, from at least one database previously unconnected with the biological diagram, with semantics extracted from at least one region on the biological diagram to which the at least one biological concept, entity (130,301), interaction or relationship relates.

8. The method of claim 7, wherein the extracted semantics are represented in a controlled, computable local format.

9. The method of claim 7, further comprising mapping at least one linked biological concept, entity (130,301) interaction or relationship on the biological diagram (100,300, 511,512,513,514,515,516) in a location from which the linked semantics were extracted, and displaying the overlaid biological diagram (100,300, 511,512,513,514,515,516) in a diagram viewer (100).

10. A method of navigating through a corpus of biological knowledge sources or databases using an interactive biological diagram (100,300, 511,512,513,514,515,516), said method comprising the steps of:
accessing the interactive biological diagram (100,300, 511,512,513,514,515,516) and
selecting at least one concept, relationship, entity (130,301) or interaction displayed in the biological diagram (100,300, 511,512,513,514,515,516);
automatically searching the corpus of biological knowledge sources or databases to locate members of the corpus that contain at least one selected concept, relationship, entity (130,301) or interaction; and
identifying the members or portions thereof which contain at least one selected concept, relationship, entity (130,301) or interaction.

11. The method of claim 10, wherein said corpus of biological knowledge sources comprises a corpus of textual data, a corpus of biological diagrams, or a corpus of experimental data.

12. The method of claim 10, wherein the corpus of biological knowledge sources is contained in multiple linked databases.

13. The method of claim 12, further comprising automatically initiating an operation in the databases, based upon at least one selected concept, relationship, entity (130,301) or interaction.

14. The method of claim 10, wherein the corpus of biological knowledge comprises a corpus of annotations, and wherein said automatic searching is performed to rapidly drill down to specific annotations where at least one selected concept, relationship, entity (130,301) or interaction occurs.

15. A method of navigating through a corpus of biological knowledge sources or databases using annotations linked to biological concepts, entities, relationships and interactions, said method comprising the steps of:
selecting at least one annotation linked to a concept, relationship, entity (130,301) or interaction;
automatically searching the corpus of biological knowledge sources or databases to locate members of the corpus that contain at least one selected annotation; and
identifying the members or portions thereof which contain at least one selected annotation.

16. The method of claim 15, wherein the corpus of biological knowledge sources comprises a corpus of biological diagrams.

17. The method of claim 16, wherein the corpus of biological knowledge sources further includes at least one data source linked to the biological diagrams.

18. A method of managing a user context tool for navigating biological information, said method comprising the steps of:
inputting at least one item represented in a biological diagram; and
representing each said item as at least one of a concept, relationship, entity (130,301), interaction or annotation in the user context.

19. The method of claim 18, wherein said inputting comprises manual selection and inputting at least one of said items into the user context.

20. The method of claim 18, wherein inputting of at least one said item is performed by an operation initiated from within an interactive biological diagram (100,300, 511,512,513,514,515,516).

21. The method of claim 18, further comprising at least one of viewing, modifying, deleting or extending at least one of said items.

22. The method of claim 21, wherein at least one of said items is viewed, modified, deleted, extended or inputted via an operation initiated within at least one of an interactive biological diagram (100,300, 511,512,513,514,515,516), textual data or experimental data.

23. A method of navigating and manipulating biological diagrams, said method comprising the steps of:
providing a user context containing at least one user context item;
identifying biological diagrams containing at least one of said user context items; and
returning results identifying said biological diagrams containing at least one of said user context items.

24. The method of claim 23, further comprising overlaying (110) at least one of said user context items on at least one of said biological diagram (100,300, 511,512,513,514,515,516) containing said at least one of said user context items.

25. The method of claim 23, further comprising at least one of searching, filtering and organizing a corpus of biological diagrams based on a number of user context items from a selected set from the user context, that each biological diagram in the corpus contains.

26. The method of claim 23, further comprising at least one of searching, filtering and organizing a corpus of linked data sources based on a number of user context items from a selected set from the user context, that each member of the linked data sources in the corpus contains.

27. A method of comparing one or more interactive biological diagrams (100,300, 511,512,513,514,515,516) with one or more graphical displays of contents from at least one database, said method comprising the steps of:
providing functional interaction between views of the one or more interactive biological diagrams (100,300, 511,512,513,514,515,516) and the one or more graphical displays;
comparing at least one corresponding concept, relationship, entity (130,301), interaction, diagrammatic motif or subgraph between at least two interactive biological diagrams (100,300, 511,512,513,514,515,516), at least one interactive biological diagram (100,300, 511,512,5 13,514,515,516) and at least one graphical display or at least two graphical displays; and
identifying a result of said comparing.

28. The method of claim 27, wherein said comparing comprises comparing for discrepancies or agreements between at least one corresponding concept, relationship, entity (130,301), interaction, diagrammatic motif or subgraph; and wherein said identifying comprises identifying at least one pair of corresponding concepts, relationships, entities, interactions, diagrammatic motifs or subgraphs that are in agreement with or disparate from each other.

29. The method of claim 27, further comprising searching and identifying at least one concept, relationship, entity (130,301), interaction, diagrammatic motif or subgraph in at least one of the interactive biological diagrams (100,300, 511,512,513,514,515,516) or graphical displays to be used as a basis for comparison in at least one other interactive biological diagram (100,300, 511,512,513,514,515,516) or graphical display; and wherein said comparing comprises at least one of searching, filtering and organizing said at least one concept, relationship, entity (130,301), interaction, diagrammatic motif or subgraph in said at least one other interactive biological diagram (100,300, 511,512,513,514,515,516) or graphical display.

30. The method of claim 27, further comprising generating a user context by converting said at least one concept, relationship, entity (130,301), interaction, diagrammatic motif or subgraph to a local format.

31. A method of searching biological data, said method comprising the steps of:
searching for and locating at least one concept, relationship, entity (130,301), interaction, diagrammatic motif or subgraph in at least one interactive biological diagram (100,300, 511,512,513,514,515,516) or graphical display;
performing at least one of searching, filtering and organizing at least one other biological diagram or biological data source, based on the at least one located concept, relationship, entity (130,301), interaction, diagrammatic motif or subgraph; and
outputting results of at least one of said searching filtering and organizing.

32. The method of claim 31, further comprising creating a user context by converting the at least one located concept, relationship, entity (130,301), interaction, diagrammatic motif or subgraph to a local format, and performing said at least one of searching, filtering and organizing based on the user context.

33. The method of claim 1, further comprising the steps of displaying multiple interactive biological diagrams (100,300, 511,512,513,514,515,516) in a single display.

34. The method of claim 33,wherein the multiple interactive biological diagrams (100,300, 511,512,513,514,515,516) are displayed adjacently, in an ordered manner.

35. The method of claim 33, further comprising simultaneously mapping at least one biological concept, entity (130,301), interaction or relationship on the multiple diagrams (100,300, 511,512,513,514,515,516) where the concept, entity (130,301), interaction or relationship appears.

36. The method of claim 22, further comprising mapping relationships between concepts, based on relationships identified in the at least one database, either within a single biological diagram (100,300, 511,512,513,514,515,516) or across multiple diagrams (100,300, 511,512,513,514,515,516).

37. A method of traversing and computing across graphical and network data structures to biological diagrams which are represented by said graphical and network data structures, said method comprising the steps of:
applying a graph theoretical technique to determine at least one of: a shortest path in a network; at least one spanning tree; degrees of connectedness; graph width; redundancy; redundant paths; alternative paths; graph traversal, identification of a subgraph, and
identification of a motif structure within a graph.

38. A method comprising forwarding a result obtained from the method of any of claims 1 through 37 to a remote location.

39. A method comprising transmitting data representing a result obtained from the method of any of claims 1 through 37 to a remote location.

40. A method comprising receiving a result obtained from a method of any of claims 1 through 37 from a remote location.

41. A system for visualizing biological data by displaying an interactive biological diagram (100,300, 511,512,513,514,515,516), said system comprising:
means for providing biological information that may be displayed by a graphical representation;
means for converting the biological information to an interactive format in which the biological information is capable of being graphically displayed; and
means for assembling the converted information; and
means for visually displaying the converted information in the form of an interactive biological diagram (100,300, 511,512,513,514,515,516).

42. The system of claim 41, further comprising means for linking at least a portion of the interactive biological diagram (100,300, 511,512,513,514,515,516) to at least one other database or source of biological knowledge.

43. The system of claim 42, further comprising means for representing linked data in a local format.

44. The system of claim 41, further comprising means for annotating the biological diagram (100,300, 511,512,513,514,515,516) or a portion thereof.

45. The system of claim 44, further comprising means for overlaying (110) annotations on the biological diagram (100,300, 511,512,513,514,515,516).

46. A system for converting a biological diagram into an interactive research tool, said system comprising:
means for extracting semantics from the biological diagram in one or more areas where the biological diagram displays biological semantic meaning; and
means for linking at least one biological concept, entity (130,301), interaction, or relationship, from at least one database previously unconnected with the biological diagram, with semantics extracted from at least one region on the biological diagram to which the at least one biological concept, entity (130,301), interaction or relationship relates.

47. The system of claim 46, wherein the extracted semantics are represented in a controlled, computable local format.

48. The system of claim 46, further comprising means for mapping at least one linked biological concept, entity (130,301) interaction or relationship on the biological diagram (100,300, 511,512,513,514,515,516) in a location from which the linked semantics were extracted, and means for displaying the overlaid biological diagram (100,300, 511,512,513,514,515,516) in a diagram viewer (100).

49. A system for navigating through a corpus of biological knowledge sources or databases using an interactive biological diagram (100,300, 511,512,513,514,515,516), said system comprising:
means for accessing the interactive biological diagram (100,300, 511,512,513,514,515,516) and selecting at least one concept, relationship, entity (130,301) or interaction displayed in the biological diagram (100,300, 511,512,513,514,515,516);
means for automatically searching the corpus of biological knowledge sources or databases to locate members of the corpus that contain at least one selected concept, relationship, entity (130,301) or interaction; and
means for identifying the members or portions thereof which contain at least one selected concept, relationship, entity (130,301) or interaction.

50. The system of claim 49, wherein the corpus of biological knowledge is contained in multiple linked databases.

51. The system of claim 49, wherein the corpus of biological knowledge sources comprises a corpus of annotations, and wherein said automatic searching is performed to rapidly drill down to specific annotations where at least one selected concept, relationship, entity (130,301) or interaction occurs.

52. A system for navigating through a corpus of biological knowledge sources or databases using annotations linked to biological concepts, entities, relationships and interactions, said system comprising:
means for selecting at least one annotation linked to a concept, relationship, entity (130,301) or interaction;
means for automatically searching the corpus of biological knowledge sources or databases to locate members of the corpus that contain at least one selected annotation; and
means for identifying the members or portions thereof which contain at least one selected annotation.

53. A system for managing a user context tool for navigating biological information, said system comprising:
means for inputting at least one item represented in a biological diagram; and
means for representing each said item as at least one of a concept, relationship, entity (130,301), interaction or annotation in the user context.

54. The system of claim 53, further comprising at least one of means for viewing, means for modifying, means for deleting and means for extending at least one of said items.

55. A system for navigating and manipulating biological diagrams, said system comprising:
means for providing a user context containing at least one user context item;
means for identifying biological diagrams containing at least one of said user context items; and
means for returning results identifying said biological diagrams containing at least one of said user context items.

56. The system of claim 55, further comprising means for overlaying (110) at least one of said user context items on at least one of said biological diagrams containing said at least one of said user context items.

57. The system of claim 55, further comprising at least one of means for searching, means for filtering and means for organizing a corpus of biological diagrams based on a number of user context items from a selected set from the user context, that each biological diagram or member of the linked data sources in the corpus contains.

58. A system for comparing one or more interactive biological diagrams (100,300, 511,512,513,514,515,516) with one or more graphical displays of contents from at least one database, said system comprising:
means for providing functional interaction between views of the one or more interactive biological diagrams (100,300, 511,512,513,514,515,516) and the one or more graphical displays;
means for comparing at least one corresponding concept, relationship, entity (130,301), interaction, diagrammatic motif or subgraph between at least two interactive biological diagrams (100,300, 511,512,513,514,515,516), at least one interactive biological diagram (100,300, 511,512,513,514,515,516) and at least one graphical display or at least two graphical displays; and
means for identifying a result of said comparing.

59. The system of claim 58, further comprising means for generating a user context by converting said at least one concept, relationship, entity (130,301), interaction, diagrammatic motif or subgraph to a local format.

60. A system for searching biological data, said system comprising:
means for searching for and locating at least one concept, relationship, entity (130,301), interaction, diagrammatic motif or subgraph in at least one interactive biological diagram or graphical display;
means for performing at least one of searching, filtering and organizing at least one other biological diagram or biological data source, based on the at least one located concept, relationship, entity (130,301), interaction, diagrammatic motif or subgraph; and
means for outputting results of at least one of said searching filtering and organizing.

61. The system of claim 60, further comprising means for creating a user context by converting the at least one located concept, relationship, entity (130,301), interaction, diagrammatic motif or subgraph to a local format, and performing said at least one of searching, filtering and organizing based on the user context.

62. The system of claim 41, further comprising means for displaying multiple interactive biological diagrams (100,300, 511,512,513,514,515,516) in a single display.

63. The system of claim 62, further comprising means for simultaneously mapping at least one biological concept, entity (130,301), interaction or relationship on the multiple diagrams (100,300, 511,512,513,514,515,516) where the concept, entity (130,301), interaction or relationship appears.

64. A system for traversing and computing across graphical and network data structures to biological diagrams which are represented by said graphical and network data structures, said system comprising:
means for applying a graph theoretical technique to determine at least one of: a shortest path in a network; at least one spanning tree; degrees of connectedness; graph width; redundancy; redundant paths; alternative paths; graph traversal, identification of a subgraph, and identification of a motif structure within a graph.

65. A tool for comparing biological knowledge extracted from text against an existing biological diagram, said tool comprising:
a text viewer into which at least a portion of a textual document may be imported and viewed;
means for text mining the at least a portion of a textual document having been imported into the text viewer;
a list-based text editor that lists entities and interactions having been identified by said means for text mining;
a diagram viewer (100) and means for importing at least a portion of an existing biological diagram into said diagram viewer (100);
means for overlaying the identified entities and interactions on said at least a portion of an existing biological diagram that is displayed in said diagram viewer (100); and
means for visually distinguishing the overlaid entities and interactions from a remainder of the displayed biological diagram.

66. The tool of claim 65, wherein the entities and interactions having been identified are represented in a local format.

67. The tool of claim 65, further comprising means for assigning directionality to the listed interactions; means for selecting interactions and associated entities in the list-based editor; and means for populating diagrammatic renderings representing said selected interactions and associated entities.

68. The tool of claim 67, further comprising means for overlaying said populated graphical renderings on an existing biological diagram displayed in said diagram viewer (100).

69. The tool of claim 68, further comprising means for converting said at least a portion of an existing biological diagram to a local format and, based on values contained in the local format, comparing said populated graphical renderings with corresponding parts of said at least a portion of an existing biological diagram.

70. The tool of claim 69, further comprising means for identifying and visually displaying discrepancies between said graphical renderings and the existing biological diagram.

71. The tool of claim 65, further comprising means for automatically searching databases of existing biological diagrams for biological diagrams which contain a user-specified set of interactions, entities, concepts and/or relationships and returning those existing biological diagrams that contain the user-specified set to the user for display in said diagram viewer (100) for use in overlaying (110) and comparing the identified entities and interactions therewith.

72. The tool of claim 68, further comprising means for annotating the existing biological diagram.

73. A computer readable medium carrying one or more sequences of instructions for visualizing biological data by displaying an interactive biological diagram (100,300, 511,512,513,514,515,516), wherein execution of one or more sequences of instructions by one or more processors causes the one or more processors to perform the steps of:
providing biological information that may be displayed by a graphical representation; converting the biological information to an interactive format in which the biological information is capable of being graphically displayed; and
assembling the converted information and visually displaying the converted information in the form of an interactive biological diagram (100,300, 511,512,513,514,515,516).

74. The computer readable medium of claim 73, wherein execution of one or more sequences of instructions by one or more processors causes the one or more processors to perform the further step of displaying multiple interactive biological diagrams (100,300, 511,512,513,514,515,516) in a single display.

75. The computer readable medium of claim 74, wherein the multiple interactive biological diagrams (100,300, 511,512,513,514,515,516) are displayed adjacently, in an ordered manner.

76. The computer readable medium of claim 75, wherein said ordered manner is selected from the group consisting of: time sequence, biological location, and experimental protocol sequence.

77. A computer readable medium carrying one or more sequences of instructions for converting a biological diagram into an interactive research tool, wherein execution of one or more sequences of instructions by one or more processors causes the one or more processors to perform the steps of:
extracting semantics from the biological diagram in one or more areas where the biological diagram displays biological semantic meaning; and
linking at least one biological concept, entity (130,301), interaction, or relationship, from at least one database previously unconnected with the biological diagram, with semantics extracted from at least one region on the biological diagram to which the at least one biological concept, entity (130,301), interaction or relationship relates.

78. A computer readable medium carrying one or more sequences of instructions for navigating through a corpus of biological knowledge sources or databases using an interactive biological diagram (100,300, 511,512,513,514,515,516), wherein execution of one or more sequences of instructions by one or more processors causes the one or more processors to perform the steps of:
accessing the interactive biological diagram (100,300, 511,512,513,514,315,316) and selecting at least one concept, relationship, entity (130,301) or interaction displayed in the biological diagram (100,300, 511,512,513,514,515,516);
automatically searching the corpus of biological knowledge sources or databases to locate members of the corpus that contain at least one selected concept, relationship, entity (130,301) or interaction; and
identifying the members or portions thereof which contain at least one selected concept, relationship, entity (130,301) or interaction.

79. A computer readable medium carrying one or more sequences of instructions for navigating through a corpus of biological knowledge sources or databases using annotations linked to biological concepts, entities, relationships and interactions, wherein execution of one or more sequences of instructions by one or more processors causes the one or more processors to perform the steps of:
selecting at least one annotation linked to a concept, relationship, entity (130,301) or interaction;
automatically searching the corpus of biological knowledge sources or databases to locate members of the corpus that contain at least one selected annotation; and
identifying the members or portions thereof which contain at least one selected annotation.

80. A computer readable medium carrying one or more sequences of instructions for managing a user context tool for navigating biological information, wherein execution of one or more sequences of instructions by one or more processors causes the one or more processors to perform the steps of:
inputting at least one item represented in a biological diagram; and
representing each said item as at least one of a concept, relationship, entity (130,301), interaction or annotation in the user context.

81. A computer readable medium carrying one or more sequences of instructions for navigating and manipulating biological diagrams, wherein execution of one or more sequences of instructions by one or more processors causes the one or more processors to perform the steps of:
providing a user context containing at least one user context item;
identifying biological diagrams containing at least one of said user context items; and
returning results identifying said biological diagrams containing at least one of said user context items.

82. A computer readable medium carrying one or more sequences of instructions for comparing one or more interactive biological diagrams (100,300, 511,512,513,514,515,516) with one or more graphical displays of contents from at least one database, wherein execution of one or more sequences of instructions by one or more processors causes the one or more processors to perform the steps of:
providing functional interaction between views of the one or more interactive biological diagrams (100,300, 511,512,513,514,515,516) and the one or more graphical displays;
comparing at least one corresponding concept, relationship, entity (130,301), interaction, diagrammatic motif or subgraph between at least two interactive biological diagrams (100,300, 511,512,513,514,515,516), at least one interactive biological diagram (100,300, 511,512,513,514,515,516) and at least one graphical display or at least two graphical displays; and
identifying a result of said comparing.

83. A computer readable medium carrying one or more sequences of instructions for searching biological data, wherein execution of one or more sequences of instructions by one or more processors causes the one or more processors to perform the steps of:
searching for and locating at least one concept, relationship, entity (130,301), interaction, diagrammatic motif or subgraph in at least one interactive biological diagram (100,300, 511,512,513,514,515,516) or graphical display;
performing at least one of searching, filtering and organizing at least one other biological diagram or biological data source, based on the at least one located concept, relationship, entity (130,301), interaction, diagrammatic motif or subgraph; and
outputting results of at least one of said searching filtering and organizing.

84. A computer readable medium carrying one or more sequences of instructions for traversing and computing across graphical and network data structures to biological diagrams which are represented by said graphical and network data structures, wherein execution of one or more sequences of instructions by one or more processors causes the one or more processors to perform the steps of:
applying a graph theoretical technique to determine at least one of: a shortest path in a network; at least one spanning tree; degrees of connectedness; graph width; redundancy; redundant paths; alternative paths; graph traversal, identification of a subgraph, and
identification of a motif structure within a graph.
